# EUROPEAN PATENT SPECIFICATION

(11) **EP 1 255 774 B1**
(45) Date of publication and mention of the grant of the patent: **14.09.2005**
(21) Application number: 01904775.2
(22) Date of filing: 16.02.2001
(51) Int. Cl.: C07K 14/435, A61K 38/17, A01K 67/027

(54) **METHODS FOR IDENTIFYING COMPOUNDS USEFUL FOR THE TREATMENT OF OBESITY INVOLVING FOXC2**
METHODEN ZUR IDENTIFIZIERUNG VON ZUSAMMENSETZUNGEN, DIE FÜR DIE BEHANDLUNG VON FETTLEIBIGKEIT NÜTZLICH SIND, UNTER VERWENDUNG VON FOXC2
METHODES D'IDENTIFICATION DE COMPOSES UTILES POUR LE TRAITEMENT D'OBESITE IMPLIQUANT LE GENE FOXC2

(30) Priority: 18.02.2000 SE 0000531; 26.05.2000 SE 0001982; 06.06.2000 US 587945; 14.12.2000 SE 0004629
(43) Date of publication of application: 13.11.2002
(73) Proprietor: LeanGene AB, 413 45 Göteborg (SE)
(72) Inventor: ENERBÄCK, Sven, S-431 96 Mölndal (SE); CARLSSON, Peter, S-448 37 Floda (SE)
(74) Representative: Becker Kurig Straus
(86) International application number: PCT/SE2001/000339
(87) International publication number: WO 2001/060853

(56) References cited:
- WO-A1-92/06104
- WO-A1-98/54216
- WO-A1-99/52415
- NAOYUKI MIURA ET AL.: 'Isolation of the mouse (MFH-1) and human (FKHL14) mesenchyme fork head-1 genes reveals conversation of their gene and protein structures' GENOMICS vol. 41, 1997, pages 489 - 492, XP000881835

### SEQUENCE LISTING

<110> Pharmacia AB
<120> Animal Model
<130> 00083
<140>
   <141>
<160> 2
<170> PatentIn ver. 2.1
<210> 1
   <211> 3289
   <212> DNA
   <213> Homo sapiens
<220>
   <221> CDS
   <222> (1197)..(2702)
<300>
   <308> GenBank/Y08223
   <309> 1997-05-14
   <313> 1 TO 3289
<300>
   <301> Miura et al.,
   <303> Genomics
   <304> 41
   <306> 489-492
   <307> 1997
<400> 1
gaattcggag gattaagttg tcagtcagca cgttgctacc ttcccctcta tgcactccgc 60
   tgcctggctc ctcggcgggg agcgagggaa actcagtttg tagggtttac ctctaaaacc 120
   tcgataggtt atccttgacg accccgagcc tggaaactcc ctgttgatga ttaattattt 180
   gattaaataa gtataacatc caggagaggc cctgccattc caatccagcg cgtttgcttt 240
   tgaatccatt acacctgggc ccccataatt aggaaatcta attattcgct tcatcactca 300
   ttaataagaa aaatgtccca ggatcattgc tacttacaag gtctttggga gagatatttt 360
   actctattaa tccattctat tttatatttc aaattgattt tttttaacag aggaaagtgg 420
   ctatcttttt gttttgggca tgtgggccca ttcaccaaaa tgtgatcata aaataaattt 480
   taataagata taacttttta aaaagttttc aagtgaagac ggagtcgccg cggaggccgg 540
   ggcggcgggg tcttagagcc gacggattcc tgcgctcctc gccccgattg gcgccggact 600
   cctctcagct gccgggtgat tggctcaaag ttccgggagg gggcgtggcc cgaggaaagt 660
   aaaaactcgc tttcagcaag aagacttttg aaacttttcc caatccctaa aagggacttg 720
   gcctcttttt ctgggctcag cggggcagcc gctcggaccc cggcgcgctg accctcgggg 780
   ctgccgattc gctgggggct tggagagcct cctgcgcccc tcctcgcgcg ggccgagggt 840
   ccaccttggt ccccaggccg cggcgtctcc gctgggtccg cggccgcccg cctgcccgcg 900
   ctgccgccgc cgggtcctgg agccagcgag gagcggggcc ggcgctgcgc ttgcccgggg 960
   cgcgccctcc aggatgccga tccgcccggt ccgctgaaag cgcgcgcccc tgctcggccc 1020
   gagcgacgac gaccgcgcac cctcgccccg gaggctgcca ggagaccggg gccgcccctc 1080
   ccgctcccct cctctccccc tctggctctc tcgcgctctc tcgctctcag ggcccccctc 1140
   gctcccccgg ccgcagtccg tgcgcgaggg cgccggcgag ccgtctcgga agcagc atg 1199
   cag gcg cgc tac tcc gtg tcc gac ccc aac gcc ctg gga gtg gtg ccc    1247
   tac ctg agc gag cag aat tac tac cgg gct gcg ggc agc tac ggc ggc    1295
   atg gcc agc ccc atg ggc gtc tat tcc ggc cac ccg gag cag tac agc    1343
   gcg ggg atg ggc cgc tcc tac gcg ccc tac cac cac cac cag ccc gcg    1391
   gcg cct aag gac ctg gtg aag ccg ccc tac agc tac atc gcg ctc atc    1439
   acc atg gcc atc cag aac gcg ccc gag aag aag atc acc ttg aac ggc    1487
   atc tac cag ttc atc atg gac cgc ttc ccc ttc tac cgg gag aac aag    1535
   cag ggc tgg cag aac agc atc cgc cac aac ctc tcg ctc aac gag tgc    1583
   ttc gtc aag gtg ccc cgc gac gac aag aag ccc ggc aag ggc agt tac    1631
   tgg acc ctg gac ccg gac tcc tac aac atg ttc gag aac ggc agc ttc    1679
   ctg cgg cgc cgg cgg cgc ttc aaa aag aag gac gtg tcc aag gag aag    1727
   gag gag cgg gcc cac ctc aag gag ccg ccc ccg gcg gcg tcc aag ggc      1775
   gcc ccg gcc acc ccc cac cta gcg gac gcc ccc aag gag gcc gag aag      1823
   aag gtg gtg atc aag agc gag gcg gcg tcc ccg gcg ctg ccg gtc atc      1871
   acc aag gtg gag acg ctg agc ccc gag agc gcg ctg cag ggc agc ccg      1919
   cgc agc gcg gcc tcc acg ccc gcc ggc tcc ccc gac ggt tcg ctg ccg      1967
   gag cac cac gcc gcg gcg ccc aac ggg ctg cct ggc ttc agc gtg gag      2015
   aac atc atg acc ctg cga acg tcg ccg ccg ggc gga gag ctg agc ccg      2063
   ggg gcc gga cgc gcg ggc ctg gtg gtg ccg ccg ctg gcg ctg cca tac      2111
   gcc gcc gcg ccg ccc gcc gcc tac ggc cag ccg tgc gct cag ggc ctg      2159
   gag gcc ggg gcc gcc ggg ggc tac cag tgc agc atg cga gcg atg agc      2207
   ctg tac acc ggg gcc gag cgg ccg gcg cac atg tgc gtc ccg ccc gcc      2255
   ctg gac gag gcc ctc tcg gac cac ccg agc ggc ccc acg tcg ccc ctg      2303
   agc gct ctc aac ctc gcc gcc ggc cag gag ggc gcg ctc gcc gcc acg      2351
   ggc cac cac cac cag cac cac ggc cac cac cac ccg cag gcg ccg ccg      2399
   ccc ccg ccg gct ccc cag ccc cag ccg acg ccg cag ccc ggg gcc gcc      2447
   gcg gcg cag gcg gcc tcc tgg tat ctc aac cac agc ggg gac ctg aac    2495
   cac ctc ccc ggc cac acg ttc gcg gcc cag cag caa act ttc ccc aac    2543
   gtg cgg gag atg ttc aac tcc cac cgg ctg ggg att gag aac tcg acc    2591
   ctc ggg gag tcc cag gtg agt ggc aat gcc agc tgc cag ctg ccc tac    2639
   aga tcc acg ccg cct ctc tat cgc cac gca gcc ccc tac tcc tac gac    2687
   tgc acg aaa tac tga cgtgtcccgg gacctcccct ccccggcccg ctccggcttc    2742
   gcttcccagc cccgacccaa ccagacaatt aaggggctgc agagacgcaa aaaagaaaca 2802
   aaacatgtcc accaaccttt tctcagaccc gggagcagag agcgggcacg ctagccccca 2862
   gccgtctgtg aagagcgcag gtaactttaa ttcgccgccc cgtttctggg atcccaggaa 2922
   acccctccaa agggacgcag cccaacaaaa tgagtattgg tcttaaaatc cccctcccct 2982
   accaggacgg ctgtgctgtg ctcgacctga gctttcaaaa gttaagttat ggacccaaat 3042
   cccatagcga gcccctagtg actttctgta ggggtcccca taggtgtatg ggggtctcta 3102
   tagataatat atgtgctgtg tgtaatttta aatttctcca accgtgctgt acaaatgtgt 3162
   ggatttgtaa tcaggctatt ttgttgttgt tgttgttgtt cagagccatt aatataatat 3222
   ttaaagttga gttcactgga taagtttttc atcttgccca accatttcta actgccaaat 3282
   tgaattc                                                           3289
<210> 2
   <211> 501
   <212> PRT
   <213> Homo sapiens
<400> 2

## Description

### TECHNICAL FIELD

The invention relates to transgenic non-human mammalian animals being capable of expressing the human *FOXC2* gene in its adipose tissue. The invention also relates to methods for identifying compounds useful for the treatment of medical conditions related to obesity or diabetes, said compounds being capable of stimulating expression of the human *FOXC2* gene, or being capable of stimulating the biological activity of a polypeptide encoded by the human *FOXC2* gene. The invention further relates to methods for identifying compounds useful for the treatment of medical conditions related to malnutrition, said compounds being capable of decreasing expression of the human *FOXC2* gene, or being capable of decreasing the biological activity of a polypeptide encoded by the human *FOXC2* gene.

### BACKGROUND ART

More than half of the men and women in the United States, 30 years of age and older, are now *considered* overweight, and nearly one-quarter are clinically obese (Wickelgren, 1998). This high prevalence has led to increases in the medical conditions that often accompany obesity, especially non-insulin dependent diabetes mellitus (NIDDM), hypertension, cardiovascular disorders, and certain cancers. Perhaps most importantly, obesity confers a significant increased rate of mortality when compared with that of individuals of normal body weight. Obesity results from a chronic imbalance between energy intake (feeding) and energy expenditure. Energy expenditure has several major components including basal metabolism, physical activity, and adaptive (nonshivering) thermogenesis. This latter process refers to energy that is dissipated in response to changing environmental conditions, most notably exposure to cold or excessive caloric intake (so-called diet-induced thermogenesis). To better understand the mechanisms that lead to obesity and to develop strategies in certain patient populations to control obesity, we need to develop a better underlying knowledge of the molecular events that regulate the differentiation of preadipocytes and stem cells to adipocytes, the major component of adipose tissue.

### Role of adipose tissue

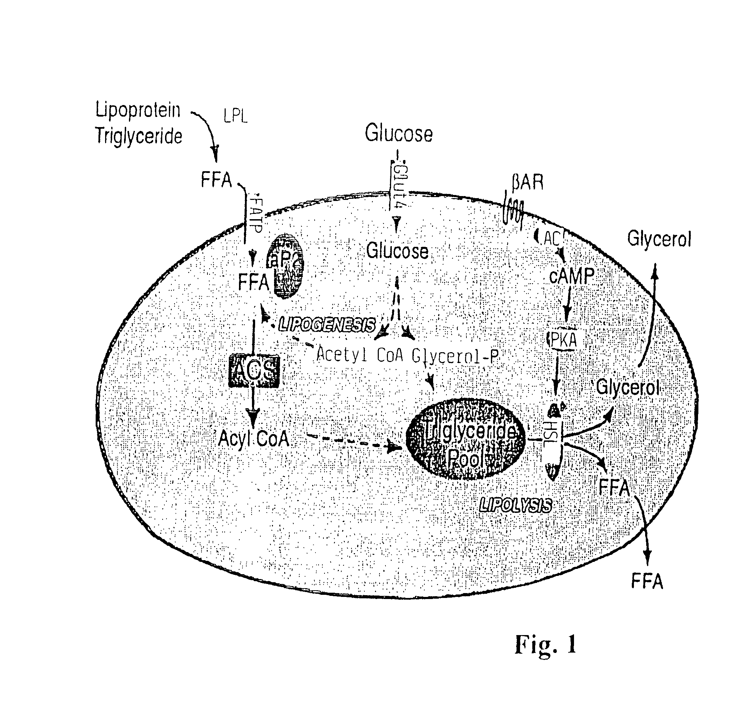

The reason for existence of the adipocyte is to store energy for use during periods of caloric insufficiency. Postprandially, dietary fat is absorbed via the intestine and secreted into the circulation as large triglyceride (TG) rich particles called chylomicrons (chylo). Lipoprotein lipase (LPL), although produced by adipocytes, is localized to the endothelial cell surface where it hydrolyses TG resulting in the release of free fatty acids (FFA). Much of these are taken up by the adipose tissue either passive or active via FFA transporters. The FFAs are then activated to an acyl CoA form and re-esterified by an enzymatic cascade to form storage TG. At the same time, glucose, which also increases in the circulation postprandially, is taken up into adipose tissue via specific plasma membrane glucose transporters. These two substrates (glucose and FFA) are the building blocks for formation of storage TG. On the other hand, during fasting, FFAs are released from the adipose tissue TG pool through the action of hormone sensitive lipase (HSL: Fig. 1). Clearly, efficient functioning of adipose tissue is dependent on the coordinated control of each of these processes and the proteins involved.

In recent years, a growing body of evidence has demonstrated a dual role for adipocytes, also being a source of numerous hormones that regulate both the adipocyte itself and many other systems within the body. Adipocytes produce leptin as a function of adipose energy stores. Leptin acts through receptors in the hypothalamus to regulate appetite, activity of brown adipose tissue (BAT), insulin secretion via sympathetic nervous system output, and important neuroendocrine adaptive responses to fasting and control of reproduction. The gene encoding leptin was identified by positional cloning (Zhang *et al*., 1994) and is the mutation leading to the profound obese phenotype of the ob/ob mouse, characterized by severe obesity, NIDDM, diminished fertility and hypothermia. The db-gene codes for a hypothalamic receptor for leptin (Chua *et al.,* 1996) and the db/db mutant mice show a similar phenotype with ob/ob mice, but here the defect lies in the block of leptin receptor downstream signaling. After leptin administration, it was possible to correct the defect only in the ob/ob, but not db/db mice as predicted by Coleman's parabiosis experiments (Coleman, 1973).

Another adipocyte product, the cytokine tumor necrosis factor α (TNFα), has profound effects on adipocyte differentiation, and energy metabolism, and can even induce adipocyte dedifferentiation and apoptosis. Furthermore, TNFα has more systemic implications as it has been shown to play a role in the genesis of insulin resistance associated with obesity (Hotamisligil *et al*., 1993). In obese humans and numerous rodent models of obesity-diabetes syndromes, there is a marked elevation in muscle and adipose TNFα production, as compared with tissues from lean individuals (Hotamisligil *et al*., 1995; Hotamisligil *et al*., 1993). TNFα levels can be reduced with weight loss (Hotamisligil *et al*., 1995) or after treatment with the insulin-sensitizing agent pioglitazone (Nofmann *et al*., 1994).

A third adipocyte product, the acylation stimulating protein (ASP) exert autocrine action on the adipocyte, having potent anabolic effects on human adipose tissue by stimulation of glucose transport and FFA esterfication (Maslowska *et al*., 1997; Walsh *et al*., 1989). ASP is generated by the interaction of complement D (identical to adipsin), factor B, and complement C3, components of the alternate complement pathway all produced by adipocytes (Choy and Spiegelman, 1996).

### White adipose tissue versus brown adipose tissue

There are two different types of adipose tissue in the body, WAT and BAT, which have quite opposite physiological functions although they both have the same "machinery" for lipogenic and lipolytic activity. WAT stores excess energy as triglycerides and releases free fatty acids in response to energy requirements at other sites. BAT on the other hand is involved in adaptive (non-shivering) thermogenesis. BAT is found only at certain sites in the body of rodent, such as in interscapular, perirenal and retroperitoneal regions. In human neonates BAT is present in large quantities but its thermogenic activity decreases shortly after birth and the tissue is gradually converted into white type adipose tissue (Lean *et al*., 1986). However, judged by expression of the brown fat specific uncoupling protein 1 (UCP1) mRNA, substantial amounts of brown adipocytes exist throughout life in human adipose deposits, which are generally classified as white (Krief *et al*., 1993).

Brown adipocytes have a multilocular disposition of fat droplets, i.e. a number of individual droplets within each adipocyte, whereas the white adipocyte has a single fat droplet within the cell. Furthermore, the brown adipocyte has a central nucleus and a large number of mitochondria in contrast to the white adipocyte, which has very few mitochondria and a nucleus that is displaced towards the plasma membrane by the lipid droplet. The only known gene marker to distinguish BAT from WAT, or any other celltypcs is the expression of UCPI in brown adipocytes. Due to the presence of this unique mitochondrial protein brown adipocytes have the ability of facultative heat production, which is highly regulated by sympathetic nerve activity. UCP1 is a proton translocator in the inner mitochondrial membrane and functions as a facultative uncoupler of the mitochondrial respiratory chain (Nicholls and Locke, 1984). Recently two new uncoupling proteins have been identified and cloned through their sequence homology with UCP1. UCP2 is found in most tissues (Fleury *et al*., 1997), while UCP3 is expressed in BAT and skeletal muscle (Boss *et al*., 1997). The respective roles for UCP2 and UCP3 in thermogenesis and energy balance of intact animals remain to be determined. That brown fat is highly important in rodents for maintaining nutritional homeostasis is predicted by the facts that the function of BAT is impaired in obese rodents (Himms-Hagen, 1989) and transgenic mice with decreased brown fat mass develop obesity (Lowell *et al*., 1993). Since BAT is much less obvious in large animals like humans, than in rodents, skeletal muscle is thought to be the site of primary importance for normally occurring adaptive thermogenesis in large animals.

Both white and brown fat are innervated under the control of the sympathetic nervous system. There are at least three pharmacologically distinct subtypes of β-adrenergic receptors (β₁, β₂, and β₃) found in adipocytes. The β₃-adrenergic receptor (β₃-AR) is the predominant subtype in adipose tissue and it mediates the effects of norepinephrine present in the sympathetic synaptic cleft during nerve stimulation of lipolysis in WAT and BAT and of thermogenesis in BAT (Giacobino, 1995). Increased lipolysis takes place primarily through the production of cAMP and the activation of hormone-sensitive lipase through phosphorylation (Fig. 1). Thermogenesis in BAT is accomplished by increased UCP1 mRNA levels through stimulation of transcription (Rehnmark *et al*., 1990; Ricquier *et al*., 1986). Uncoupled respiration is also thought to be stimulated by increased lipolysis and the raise in intracellular concentration of FFA (Jezek *et al*., 1994). Sympathetic stimulation of brown fat also contributes to regulation of energy expenditure by increasing mitochondrial biogenesis (Wu *et al*., 1999a) and hyperplasia of brown adipocytes. In rodents, β₃-adrenergic receptors (β₃-ARs) are abundant in WAT and BAT *(Granneman et al*., 1991; Muzzin *et al*., 1991; Nahmias *et al*., 1991), white in humans, β₃-AR mRNA is abundant in BAT only, with much less or no β₃-AR mRNA found in WAT (Granneman and Lahners, 1994; Krief *et al*., 1993). Long-term treatment of obese rodents with β₃-selective agonists reduces fat stores and improves obesity-induced insulin resistance (Bloom *et al*., 1992; *Cawthorne et al*., 1992: Holloway *et al*., 1992). Thus, β₃-selective agonists are promising anti-obesity compounds. Trials of β₃-AR agonist treatment, aimed at stimulating BAT in humans have proved disappointing with respect to weight loss (Arch and Wilson, 1996). The true potential of β₃-AR agonists in humans can only be evaluated when a compound with good selectivity and efficacy at the human β₃-AR, coupled with a long duration of action *in vivo*, has been identified, however those compounds that have been evaluated in humans so far have much lower efficacy at the human than the rodent receptor. This could be explained by the fact that human and mouse/rat β₃-AR show a ~80% similarity in their amino acid sequence. Several of the β₃-AR-selective agonists (e.g. BRL 37344 and CL 316,243) have been shown to he extremely potent against mouse and rat β₃-AR but with a greatly reduced activity against the human β₃-AR. Presently, recombinant cell lines expressing human β₃-ARs are being used to identify compounds with an increased potency against the human receptor (Ito *et al*., 1998). Mice with targeted mutagenesis of the β₃-AR gene show only a modest tendency to become obese and their brown fat response to cold exposure works perfectly normal (Susulic *et al*., 1995). Deficient mice displayed an up-regulation of β₁-AR mRNA levels in both white and brown fat which most probably is the reason of the mild phenotype. These results implicate that it is possible that β₁- and β₂-ARs also play important roles in innervation of adipose tissue. Moreover, other species, including humans, have higher levels of β₁-and β₂-ARs, then β₃-AR, in adipose tissue (Lafontan and Berlan, 1993).

### Adipocyte differentiation

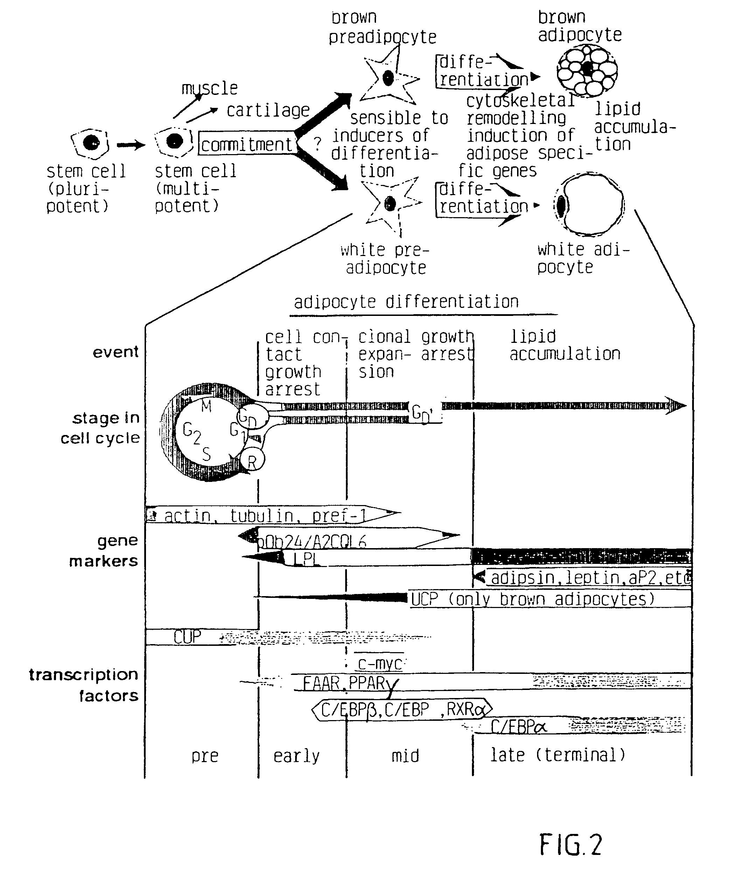

There has been some great progress during the past few years in the understanding of the adipocyte differentiation program. Most of the work leading to this understanding has been carried out using white preadipose cell lines in culture, notably the C3H10T½ and NIH 3T3 fibroblastic cell lines and the 3T3-L1 and 3T3-F442A preadipocyte cell lines. Treatment of multipotent C3H10T½ cells with 5-azacytidine (a demethylating agent) gives rise to cells committed to the myogenic, adipogenic, osteoblastic, or chondrogenic lineages. This is consistent with the view that the adipose lineage arises from the same multipotent stem cell population of mesodermal origin that gives rise to the muscle and cartilage lineages (Cornelius *et al*., 1994). Under appropriate hormonal control (e.g. glucocorticoid, insulin-like growth factor-1, and cyclic AMP or factors that mimic these agents) or experimental manipulation white preadipose cell lines are capable to differentiate into mature white adipocytes (Ailhaud *et al*., 1992). Several transcription factors have been identified, which act co-operatively and sequentially to trigger the functional differentiation program (Fig. 2).

### Transcriptional control of adipocyte differentiation through PPARs C/EBPs, and ADD1/SREBP1

Peroxisome proliferator-activated receptors (PPARs) are a class of the nuclear hormone receptors. The member PPARγ is now well recognized as serving an important role in the regulation of adipogenesis. Through the use of alternate promoters, the gene encoding PPARγ gives rise to two separate products, PPARγ1 and PPARγ2, the latter containing an additional 28 N-terminal amino acids that are reported to enhance ligand binding (Fajas *et al*., 1997; Werman *et al*., 1997). Reports that ligand activation of retrovirally expressed PPARγ2 in non-differentiating NIH-3T3 cells potently promoted adipocyte differentiation provided the most compelling evidence for the adipogenic nature of PPARγ2 (Tontonoz *et al*., 1994b). One live-born PPARγ deficient mouse has been produced and it displayed a total absence of WAT and BAT (complete lipodystrophy) and fatty liver, secondary to lipodystrophy (Barak *et al*., 1999).

Members of the PPAR family specifically function as heterodimers with the retinoid X receptor (RXR) through interactions with peroxisome proliferator response elements (PPREs) on target genes, including lipoprotein lipase (LPL; Schoonjans *et al*., 1996), the adipocyte fatty acid-binding protein 422/aP2 (Tontonoz *et al*., 1994a), phosphoenolpyruvate carboxykinase (PEPCK; Tontonoz *et al*., 1995), and stearocyl-CoA desaturase I (Miller and Ntambi, 1996). Transcriptional activity of PPARγ is induced following binding of either synthetic or naturally occurring ligands, including prostaglandins of the D2 and J2 series, with the 15-deoxy-Δ 12, 14-prostaglandin J2 derivate emerging as one of the most potent (Forman *et al*., 1995). Synthetic ligands that activate PPARγ include carbacyclin and a new class of antidiabetic drugs, the thiazolidinediones (TZDs) (Lehmann *et al*., 1995). TZDs promote adipogenesis in culture and improve insulin sensitivity *in vivo*. PPARγ activators probably modify the production of adipocyte-derived mediators of insulin resistance, such as free fatty acids or TNFα. PPARγ activation will decrease production of TNFα by adipocytes and interfere with its inhibitory effect on insulin signaling (Peraldi *et al*., 1997).

In addition, because of its tissue selective effects on genes involved in fatty acid uptake, PPARγ activation will induce repartitioning of fatty acids in the body, with enhanced accumulation of fatty acids in adipose tissue at the expense of a relative depletion of muscle fatty acids (Martin *et al*., 1997). The relative lipid depletion of muscle cells will improve their glucose metabolism and result in an improvement in insulin sensitivity. Furthermore, PPARγ decreases the expression of the adipocyte-derived signaling molecule leptin, which results in an increase in energy intake and optimization of energy usage, contributing further to PPARγ's adipogenic effect (De Vos *et al*., 1996). Recently it has been demonstrated that interaction with a novel cofactor PPARγ coactivator (PGC-1), could enhance PPARγ transcriptional activity in brown adipose tissue (Puiaserver *et al*., 1998).

Three members of the CCAAT/enhancer-binding protein (C/EBP) family of transcription factors, i.e. C/EBPα, C/EBPβ, and C/EBPδ, have been implicated in the induction of adipocyte differentiation. The factors are proteins of the bZIP class, with a basic domain that mediates DNA binding and a leucine zipper dimerization domain. Cyclic AMP and adipogenic hormones such as glucocorticoids and insulin induce a transient increase in the expression of C/EBPβ and δ early in adipocyte differentiation (Cao *et al*., 1991; MacDougald *et al*., 1994; Yeh *et al*., 1995). C/EBPβ, in synergy with C/EBPδ, then induces PPARγ expression in the preadipocyte (Wu *et al*., 1996, Wu *et al*., 1995). Mice lacking the C/EBPβ, and C/EBPδ gene have normal expression of C/EBPα and PPARγ, but this co-expression of C/EBPα and PPARγ is not sufficient for complete adipocyte differentiation in the absence of C/EBPβ and C/EBPδ (Tanaka *et al*., 1997). C/EBPα seems to play an important part in the later stages of differentiation by maintaining the differentiated adipocyte phenotype through autoactivation of its own gene (Lin and Lane, 1992; Lin and Lane, 1994). C/EBPα activates several adipocyte-specific genes such as the insulin-responsive glucose transporter-4 (GLUT4) (Kaestner *et al*., 1990). 422/aP2 (Christy *el al*., 1989), UCP (Yubero *et al*., 1994), and also the insulin receptor gene, and insulin receptor substrate I (IRS-1) (Wu *el al*., 1999b). Definitive proof that C/EBPα is required for adipocyte differentiation was obtained by showing that expression of antisense C/EBPα RNA in 3T3-L1 preadipocytes prevented differentiation (Samuelsson *et al*., 1991). Consistent with this finding, disruption of the C/EBPα gene gave rise to mice that failed to develop white adipose tissue (Wang *el al*., 1995). Taken together these findings proved that C/EBPα is both required and sufficient to induce adipocyte differentiation. The expression of C/EBPα, as well as other adipocyte genes, is induced upon ligand activation of PPARγ. Through a positive feedback loop, C/EBPα maintains the expression of PPARγ. C/EBPα and PPARγ cooperate to promote adipocyte differentiation, including adipocyte gene expression and insulin sensitivity (Wu *et al*., 1999b). It is possible that C/EBPα is ultimately an important, indirect target of the antidiabetic actions of the TZDs.

ADD1/SREBP1 (adipocyte determination and differentiation-dependent factor l/sterol regulatory element binding protein 1) is a member of the basic helix-loop-helix (bHLH) class of transcription factors. In the inactive state, the protein is membrane-bound to the endoplasmic reticulum. Upon activation (such as a low cholesterol state), ADD1/SREBP1 is proteolytically cleaved and the soluble form becomes translocated to the nucleus where it binds one of two different response elements, namely the E box and the sterol regulatory element (SRE; Brown and Goldstein, 1997). The expression of ADD1/SREBP1 is induced during differentiation of adipocytes, where it activates transcription of target genes involved in both cholesterol metabolism and fatty acid metabolism (Kim and Spiegelman, 1996). ADD1/SREBP1 potentiates the transcriptional activity of PPARγ probably through the production of endogenous ligands for PPARγ (Kim *et al*., 1998) and also by binding to and inducing the PPARγ promoter (Fajas *et al*., 1999).

When preadipocytes differentiate into adipocytes, several differentiation-linked genes are activated. Lipoprotein lipase (LPL) is one of the first genes induced during this process (Fig. 2). Two cis-regulatory elements important for gradual activation of the LPL gene during adipocyte development *in vitro* have been delimited (Enerback *et al*., 1992). These elements, LP-α and LP-β, contained a striking similarity to a consensus sequence known to bind transcription factors of the winged helix family. Results of gel mobility shift assays and DNase I and exonuclease III *in vitro* protection assays indicated that factors with DNA-binding properties similar to those of the winged helix family of transcription factors are present in adipocytes and interact with LP-α and LP-β. There is a need for identifying human winged helix genes that could be responsible for the induction of the LPL promoter and possibly regulating expression of other adipocyte specific genes.

### "Fork head" and "winged helix" genes

The "fork head" domain is an evolutionary conserved DNA-binding domain of 100 amino acids, which emerged from a sequence comparison of the transcription factor HNF-3α of rat and the homeotic *gene fork head* of *Drosophila.* X-ray crystallography of the fork head domain from HNF-3γ revealed a three-dimensional structure, the "winged helix", in which two loops (wings) are connected on the C-terminal side of the helix-loop-helix (for reviews, see Brennan, R.G. (1993) Cell 74, 773-776; Lai, E. et al. (1993) Proc. Natl. Acad. Sci. U.S.A. 90, 10421-10423).

The isolation of the mouse mesenchyme forkhead-1 (MFH-1) and the corresponding human (*FKHL14*) chromosomal genes is disclosed by Miura, N. et al. (1993) FEBS letters 326: 171-176; and (1997) Genomics 41: 489-492. The nucleotide sequences of the mouse MFH-1 gene and the human *FKHL14* gene have been deposited with the EMBL/GenBank Data Libraries under accession Nos. Y08222 (SEQ ID NO: 5) and Y08223 (SEQ ID NO: 8), respectively. A corresponding gene has been identified in *Gallus gallus* (GenBank accession numbers U37273 and U95823).

The International Patent Application WO 98/54216 discloses a gene encoding a Forkhead-Related Activator (FREAC)-11 (also known as S12), which is identical with the polypeptide encoded by the human *FKHL14* gene disclosed by Miura, *supra*.

The nomenclature for the winged helix / forkhead transcription factors has been standardized and Fox (Forkhead Box) has been adopted as the unified symbol (Kaestner et al. (2000) Genes & Development 14: 142-146; see also *htpp:*//*www.biology.pomona.edu*/*fox*). It has been agreed that the genes previously designated MFH-1 and *FKHL14* (as well as FREAC-11 and S 12) should be designated *FOXC2*.

### DESCRIPTION OF THE DRAWINGS

### FIGURE 1

Schematic view of lipogenic and lipolytic actions in the adipocyte. Triglycerides are hydrolyzed to glycerol and free fatty acids by the action of lipoprotein lipase. FFAs are transported into the adipocyte via FATP. FFAs are combined with acetyl CoA to produce acyl CoA, which is re-esterfied to triglycerides. The multi-enzyme complex hormone sensitive lipase hydrolyses triglycerides into FFAs and glycerol. FFAs can either be re-esterfied again or be released into the circulation. LPL, lipoprotein lipase; FFA, free fatty acid: FATP, fatty acid transport protein; aP2, adipocyte fatty acid-binding protein 422/aP2; ACS, acyl CoA synthetase; Glut4, glucose transporter IV; βAR, β-adrenergic receptor; AC, adenylate cyclase; PKA, protein kinase A; HSL, hormone sensitive lipase. Figure is adapted from Sethi and Hotamisligil, 1999.

### FIGURE 2

Summary of different stages and events during *in vitro* adipocyte differentiation. Our current understanding of adipocyte differentiation indicates that a pluripotent stem cell precursor gives rise to a mesenchymal precursor cell (multipotent) with the potential to differentiate along mesoderma linages of myoblast, chondroblast, osteoblast, and adipocyte. Given appropriate environmental and gene expression cues, preadipocytes undergo clonal expansion and subsequent terminal differentiation. Selected molecular events accompanying this process are indicated above. Pref-1, preadipocyte factor 1; pOb24/A2COL6, α2 chain of type VI collagen; LPL, lipoprotein lipase; aP2, adipocyte fatty acid-binding protein 422/aP2; UCP, uncoupling protein; CUP, C/EBPα undifferentiated protein; FAAR, fatty acid-activated receptor; PPARγ, peroxisome proliferator-activated receptor γ; C/EBP, CCAAT/enhancer binding protein; RXR, retinoid X receptor. Figure is adapted from Klaus, 1997.

### FIGURE 3

Northern blot analysis. Adult human tissues **(a)** and wt adult mouse tissues **(b).** The blots were analysed with probes specific (with no cross-reactivity) for *FOXC2* (human) and *FoxC2* (mouse), respectively. The experiments were carried out with 20 µg of total RNA/lane using β-actin as an internal control. Mouse adipose tissue (c) was treated with collagenase (see under "Methods"), RNA from the adipocyte fraction (Adip F) and the stroma/vascular fraction (SVF) were used and probed for *FoxC2*, LPL and GAPDH (control). In (d) 3T3-LI cells were differentiated to adipocytes in culture and then incubated for 6 hours in the absence (Basal) or presence of forskolin (Forsk.; 100 µM), 12-O-tetradecanoylphorbol-13-acetat (TPA; 100 nM), Ca²⁺ ionophore (A23187; 0.5 µM) alone or in combination as indicated. Northern blot analysis was performed and the *FOXC2* mRNA signal was assessed by β-scintillation counting in a dot-matrix β-counter. Image shows counts over the filter plotted in gray-scale intensity. Bars above the Northern blot represent actual cpm over mean cpm of controls subtracted of background cpm given as "fold induction".

### FIGURE 4

Schematic view of *FOXC2* transgene construct and Southern blot detection of integration positive mice. A 2.1-kb fragment containing the *FOXC2* cDNA was ligated downstream of the 5.4-kb aP2 enhancer/promoter region (A). Prior to pronuclear injection the transgene construct was liberated from the plasmid as an 8.9-kb *Not*I/*Age*I fragment. A 1.7-kb fragment of the aP2 promoter was used as a probe in Southern blot analysis for identification of integration positive mice. Panel B shows the identification of the three founders investigated further.

### FIGURE 5

(a): Weights of intra-abdominal WAT and interscapular BAT depots from tg mice (founder lines Tg-A. Tg-B and Tg-C) compared to that of wt mice. (b): Ratios between weights of intra-abdominal WAT to interscapular BAT for wt and tg founder lines A, B and C. Values are means ±SEM. n=4 in each group. (c): Expression levels of the *FOXC2* transgene in white adipose tissue (WAT) and brown adipose tissue (BAT) of wild-type mice (WT) and *FOXC2* transgenic mice (founder A, B, and C) as measured by Northern blot analysis (12 µg of total RNA/lane). GAPDH was used as a control.

### FIGURE 6

A 5-months-old wild-type female and a *FOXC2* transgenic female littermate. A, C, and E show WT: B. D. and F show transgenic littermate. (A, B) An exposed dorsal view of the interscapular brown fat pads, illustrating an increased size of the depot in the transgenic mouse. (C. D) An exposed ventral view, illustrating a reduction in size and change in appearance of the intrabdominal white fat pads in the transgenic mouse. (E, F) The interscapular brown fat pads and the intrabdominal white fat pads have been dissected out. BAT at the top and WAT at the bottom.

### FIGURE 7

Histologic sections of brown fat (A, B), and white fat (C, D) from a 5-months-old wild-type mouse (left) and a *FOXC2* transgenic littermate (founder A) (right). (A, B) Interscapular brown fat of the transgenic mouse consists of markedly enlarged adipocytes containing large unilocular fat droplets. (C, D) Adipocytes in white fat of the transgenic mouse show heterogeneity of size. This is in contrast to the WAT from a wild-type mouse. which consists of adipocytes of uniform size filled with a large. unilocular vacuole. In a cold adaptation experiment, mice were kept at either room temperature (RT) or at 4°C for 24 hours. Whereas there is no apparent difference between BAT from wt mice (E, G), the number and size of lipid droplets present in tg BAT at room temperature is clearly reduced when exposed to 4°C (F, H). Scale bar depicted in G equals 100 µm.

### FIGURE 8

Change of size of fat depots in *FOXC2* transgenic mice (founder C). (A) Weight comparison (expressed as percent of total body weight) for intrabdominal fat depots and interscapular brown fat depots of 5-months-old wild-type females and *FOXC2* transgenic littermates. Changes are significant, P<0.005. (B) Ratio between weights of the intrabdominal fat depot and the interscapular brown fat depot. Change is significant, P<0.0005. (C) No significant difference in body weight could be detected between the two groups. (D) No significant difference in food consumption was noticed when measured during a time period of two months. Data are means ±SD, n=3 for both WT and founder C in A-C, n=4 for both WT and founder C in D.

### FIGURE 9

Amount of different mRNAs in white adipose tissue (WAT) and brown adipose tissue (BAT) of wild-type mice (WT) and *FOXC2* transgenic mice (founder A, B, and C) as measured by Northern blot analysis (12 µg of total RNA/lane). GAPDH was used as a control for ensuring equal loading on all blots.

### FIGURE 10

Serum triglyceride is lowered in *FOXC2* transgenic mice (founder A). Serum triglyceride content was analyzed for 14-weeks-otd wild-type males and *FOXC2* transgenic littermates fed ad libitum. Change is significant, P<0.005. Data are means ±SD, n=4 for both WT and founder A.

### FIGURE 11

Total body lipid content is lowered in *FOXC2* transgenic mice (founder A). Total body lipid content was analyzed for 6-months-old wild-type males and *FOXC2* transgenic littermates fed ad libitum. Change is significant, P<0.0005. Data are means ±SD; WT, n=3 and founder A, n=4.

### FIGURE 12

Blood glucose is lowered (A) and glucose elimination is more efficient (B) in *FOXC2* transgenic mice (founder A). (A) Blood glucose levels were analyzed for 10-weeks-old wild-type mice and *FOXC2* transgenic littermates fed ad libitum. A significant (P<0.05) reduction of blood glucose levels were seen for *FOXC2* transgenic mice. (B) An intravenous glucose tolerance test was carried out on the mice used in (A). Blood samples were taken immediately before and at 1,5,20, and 50 min after intravenous injection of glucose (1 g/kg). The values were significantly changed at 0 min (P<0.05), 20 min (P<0.001), and at 50 min (P<0.05). Data are means ±SD; n=10 for both WT and founder A.

### FIGURE 13

Plasma insulin is lowered in *FOXC2* transgenic mice (A), still levels raised higher than in wild-type after intravenous (iv) glucose load (B, C) (founder A). (A) Plasma insulin levels were analyzed for 10-weeks-old wild-type mice and *FOXC2* transgenic littermates fed ad libitum. A significant (P<0.05) reduction of plasma insulin levels was seen for *FOXC2* transgenic mice. (B) An intravenous glucose tolerance test was carried out on the mice used in (A) Blood samples were taken immediately before and at 1, 5, 20. and 50 min after intravenous injection of glucose (1 g/kg). The values were significantly changed at 0 min (P<0.05), 20 min (P<0.01), and at 50 min (P<0.05). (C) Fold induction of plasma insulin levels one minute after i.v, glucose load. Change is significant, P<0.005. Data are means ±SD; n=10 for both WT and founder A.

### FIGURE 14

Hypothetical action of *FOXC2* in adipocytes. Filled arrow indicates known positive transcriptional regulation. Open arrow represents a proposed action of *FKHL14*. ADD1/SREBP1 both activates transcription of acetyl CoA carboxylase (Lopez et al., 1996), fatty acid synthase (FAS), and lipoprotein lipase (LPL), and increases the transcriptional activity of PPARγ (Fajas et al., 1999). PPARγ activates transcription of fatty acid transport protein (FATP), acyl-CoA synthetase genes (ACS: Martin et al., 1997), adipocyte fatty acid-binding protein 422/aP2 (aP2; Tontonoz et al., 1994a), LPL (Schoonjans et al., 1996), and phosphoenolpyruvate carboxykinase (PEPCK; Tontonoz et al., 1995). C/EBPα activates insulin-responsive glucose transporter-4 (GLUT4; Kaestner et al., 1990), aP2 (Christy et al., 1989), uncoupling protein I (UCP1; Yubero et al., 1994), the insulin receptor (InsR), insulin receptor substrate-1 (IRS-1; Wu et al., 1999b), and PEPCK (Park et al., 1990). A positive feedback loop between C/EBPα and PPARγ have been suggested (Wu et al., 1999b).

### FIGURE 15

Reduction in weight gain in (transgenic) tg mice. Analysis were performed on tg-A mice with wt littermates as controls, fed *ad libitum,* mice were approximately 4-6 months of age. There is a reduction in diet induced weight gains in *FOXC2tg* mice, both in females (p<0.02; a) and males (p<0.03; b), as compared with wt mice, values are means ±SEM, n=4 in each group. Mice were on a high fat diet (58.0% on a caloric basis) for seven weeks (see Methods).

### FIGURE 16

Metabolic profile. Analysis were performed on tg-A mice with wt littermates as controls, mice were approximately 4-6 months of age and fed *ad libitum*, values are means ±SEM. **a**, Total body lipid content was analysed as total lipid content of carcasses (see Methods), n=4 in each group, p<0.0004. b, Serum triglyceride is lowered in *FOXC2*tg mice, n=4 in each group, p<0.004. **c**, Serum cholesterol, no significant difference between wt and tg mice, n=4 in each group, **d**, Plasma free fatty acids (FFA) is lowered in tg mice as compared with wt littermates, n=4 in each group, p<0.02. **e,** Non-fasting plasma glucose is lowered in *FOXC2* tg mice, n=20 in each group, p<0.01. f, Plasma insulin is lowered in tg mice, analysis was performed on the same animals as in e, p<0.001. There is a reduction in diet induced weight gains in *FOXC2*tg (n=7) mice as compared with wt (n=9; p<0.01; g). Whereas the body lipid content after a high fat diet (h) increases for both wt and tg mice, as compared with mice on a standard diet (a), we note a reduction for tg (n=4) mice as compared with wt (n=6; p<0.0001) in **h.** The weight gain to grams of eaten food ratio was calculated for wt (n=10) and tg (n=9) mice on a control diet (i), no significant differences could be detected. In the corresponding experiment, with mice on a high fat diet, there is a 30% reduction (p<0.01) for tg (n=7) as compared wt (n=9) mice (i). No gender differences were observed.

### FIGURE 17

Intravenous glucose tolerance test. Wt and tg-A mice were fed a control (a, b) or high fat (c, d) diet for 14 weeks (for details see under "Methods"). After intravenous (i.v.) injection of glucose (1 g/kg), blood samples were drawn immediately before and at 1, 5, 20, 50 and 75min. Plasma glucose levels differ significantly between wt and tg mice both on a control (a, tg n=8, wt n=9; p<0.02) as well as on a high fat diet (c, tg=5, wt=6; p<0.001). Plasma insulin values differ also significantly between wt and tg mice both on a control diet (b, p<0.001) and a high fat diet (d, p<0.001). Values are means ±SEM, no gender differences were observed.

### FIGURE 18

Altered β-adrenergic sensitivity, PKA isozyme composition and activation kinetics in adipose tissue from *FOXC2* tg-A mice. (a) Left panel: CAT reporter activity directed from the RIα promoter transfected together with *FOXC2* expression vector or control vector into 3T3-L 1 pre-adipocytes. Data (n=3 experiments) are CAT values normalised for transfection efficiency, expressed as fold induction. Right panel: RIα and GAPDH mRNA levels in WAT from wild type (wt) and the three founder strains (A, B. C) over-expressing *FOXC2*. (b) Levels of RIα and RIIβ immunoreactive protein in WAT and BAT from transgenic animals (n=4) compared to wild type litter mates (expressed relative to wild type levels). (c) Kinase activities using Kemptide as substrate in the presence (total. open bars) or absence (free, solid bars) of 5 µM cAMP. Activities in transgenic WAT and BAT (n=4) are shown relative to those in wild type litter mates. (d) Kinase activities towards Kemptide measured in WAT homogenates from transgenic and wild type littermate mice incubated with increasing concentrations of cAMP. Data are representative of 2 pairs of animals analysed. Total cAMP-inducible activity was set to 100%. Right panel, immunoblot demonstrating WAT levels of RIα and RIIβ. Intracellular cAMP levels in adipocyte suspensions prepared from WAT of transgenic animals and wild type littermates (each pooled from 3 animals) and stimulated with either (e) a non-selective β-agonist (isoproterenol, I µM) or (f) a β3-selective agonist (CGP-12177A, 1 µM). Equal amounts of cells were withdrawn at different time points (0 to 10 min), mixed with stop buffer and flash-frozen. Mean ± half range (SEM) of duplicate determinations are shown.

### DISCLOSURE OF THE INVENTION

According to the present invention, the human transcription factor gene *FOXC2* is identified as a key regulator of adipocyte metabolism. Increased *FOXC2* expression, in white (WAT) and brown adipose tissue (BAT), has a pleiotropic effect on gene expression, which leads to resistance to diet induced weight gain and a decrease in: total body lipid content, serum triglycerides, plasma levels of free fatty acids, glucose and insulin. To our knowledge, *FOXC2* is the hitherto only identified gene that, in a concerted action, can counteract most, if not all, of the symptoms associated with obesity, including hypertriglyceridemia and insulin resistance; a likely consequence hereof would be protection against type 2 diabetes.

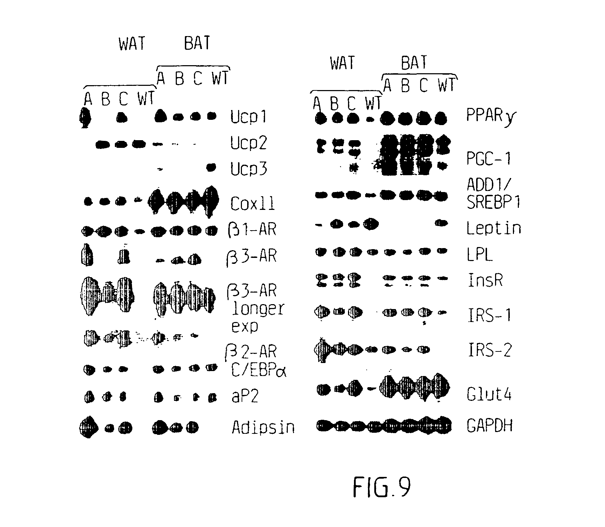

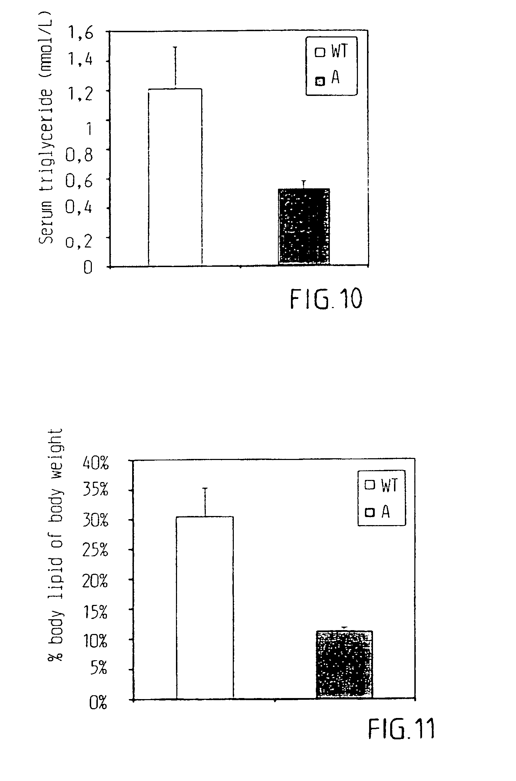

During adulthood, the human winged helix gene *FOXC2* is expressed exclusively in adipose tissue. The LPL mRNA levels in the *FOXC2* transgenic mice seem to be slightly elevated (Fig. 9), which is in concordance with the initial findings that the two winged helix cis-regulatory elements are responsible for the inducibility of the LPL promoter (Enerback *et al*., 1992). The higher expression level of LPL most probably is responsible for the significantly decreased plasma TG levels noticed in *FOXC2* transgenic mice (Fig. 10), in addition to the fact that the profound up-regulation of adipsin in both WAT and BAT (Fig. 9) most certainly is of great importance. Adipsin is a secreted protein necessary for the formation of acylation stimulating protein (ASP), which has potent anabolic effects on human adipose tissue for both glucose and free fatty acid (FFA) storage (Cianflone *et al*., 1995).

The extensive alternations seen in the *FOXC2* transgenic mice presented here predicts a very central and important role for this winged helix gene hitherto unknown to participate in molecular events in adipose tissue. It has been demonstrated by gene targeting experiments that the mouse homologue, *Mfh1*, plays a crucial role during embryonic development (Iida *et al*., 1997; Winnier *et al*., 1997), but so far nothing has been published about its function in adult mice. In a recent publication an *Mfh1*/*Pax1* double mutant was shown to be totally absent of BAT at day 15.5 dpc (Furumoto *et al*., 1999).

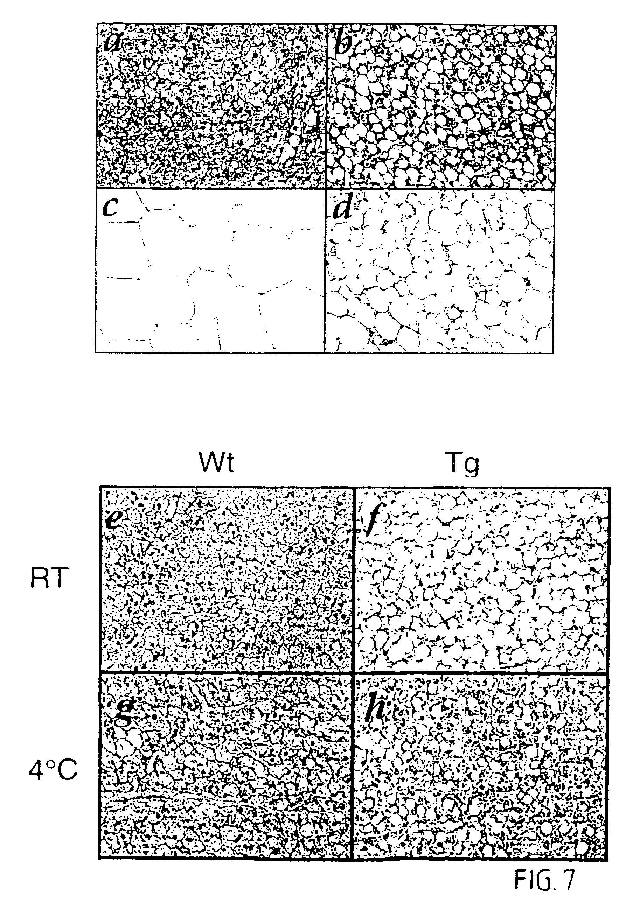

The *FOXC2* transgenic mice had a clear reduction in white adipose tissue mass. The reduced size of the white fat depots might be due solely to the reduction in size of the adipocytes (Fig. 7), but one cannot rule out the possibility that there is also a reduction in adipocyte number. There are a number of possible reasons why the *FOXC2* transgenic mice have decreased size of white adipocytes. One might presume an increased lipolytic activity of their adipocytes in general; coupled with the greater mass of brown fat this may lead to increased energy expenditure through heat production by BAT giving rise to the lean phenotype. The upregulation of β₃-AR seen in transgenic WAT (Fig. 9) will result in an elevated activation of HSL hence an increase in lipolysis (Fig. 1) ultimately leading to reduced lipid storing.

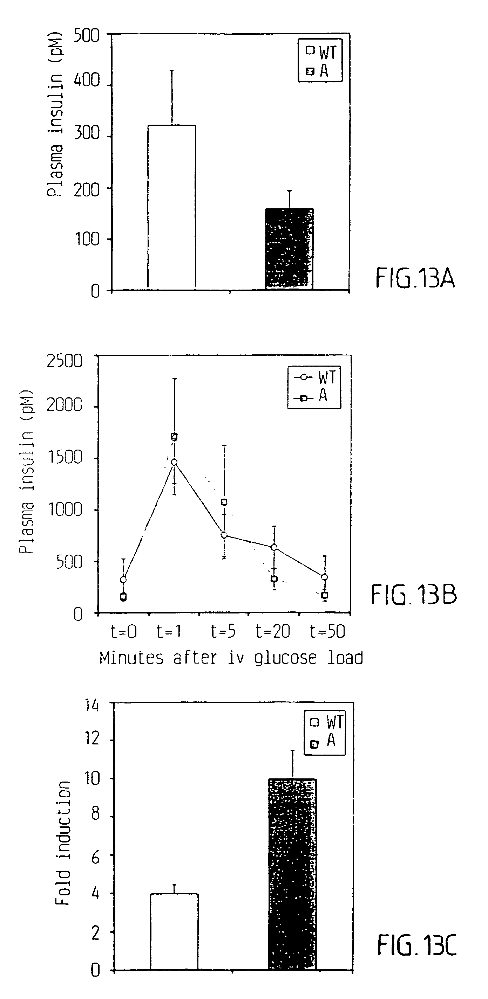

Furthermore, the transgenic mice were insulin sensitive (Fig. 13); this could be explained by both the upregulation of genes involved in insulin action (lnsR, IRS-I, IRS-2, and GLUT4; Fig. 9) and the lean body composition (Fig. 11). Insulin has a critical role in lipid metabolism, promoting the storage of triglycerides in adipocytes through numerous actions on this cell. Among these are stimulation of glucose uptake and inhibition of lipolysis, which occur very rapidly through insulin-responsive glucose transporter protein (GLUT 4) translocation or covalent modification of HSL. respectively. Insulin also stimulates fatty acid and triglyceride synthesis, through the induction of key lipogenic enzymes and induction of lipoprotein lipase. The data presented here are compatible with an increased energy turnover in the adipocytes derived from *FOXC2* transgenic mice.

The white fat depots of *FOXC2* transgenic mice had an ectopic expression of the brown fat specific marker UCP1 (Fig. 9). The origin of multilocular adipocytes in transgenic WAT (Fig. 7d) remains an enigma. It has been suggested that there is a pool of intraconveritble cells or small brown preadipocytes present in WAT. Studies on both rat and mice have demonstrated atypical occurrence of UCP1 in certain WAT depots previously thought to contain only white adipocytes (Cousin *et al*., 1992; Loncar, 1991). If submitted to cold or to treatment with a β₃-AR selective agonist, UCP1 expression was increased in WAT as in typical BAT and on histological sections one could identify small multilocular cells interspersed between the white adipocytes, which were shown to contain UCP1 by immunhistochemistry (Cousin *et al*., 1992; Ghorbani *et al*., 1997).

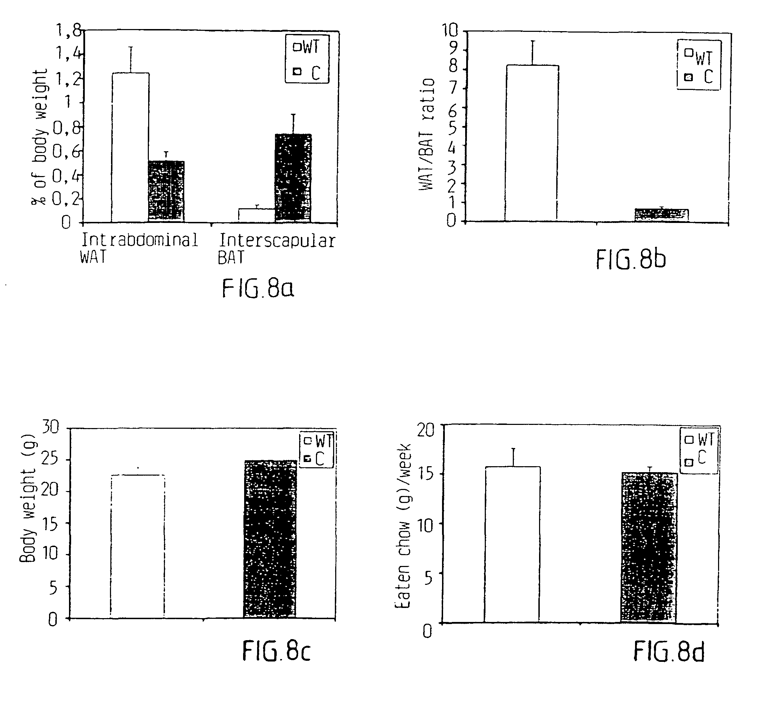

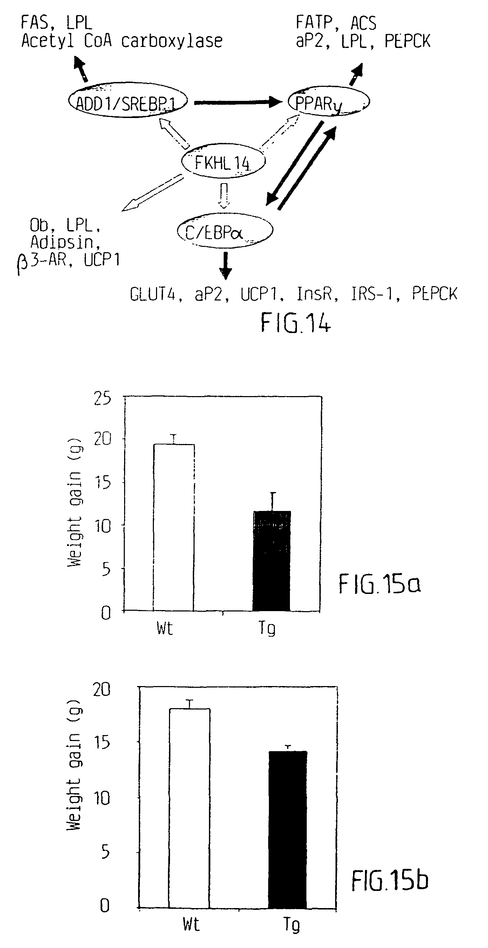

Furthermore, both UCP1 and β₃-AR mRNAs have been detected in white fat depots of human beings (Krief *et al*., 1913) and recently, it has been shown that cultures of human adipocytes derived from white fat depots express UCP1 after treatment with β₃-AR agonists (Champigny and Ricquier, 1996). Moreover, transgenic mice overexpressing β₁-AR in WAT and BAT have abundant appearance of brown fat cells in subcutaneous WAT (Soloveva *et al*., 1997). We would like to speculate that these suggested intraconveritble cells or small brown preadipocytes have undergone proliferation in our transgenic mice, readily detectable on histological sections as small multilocular cells (Fig. 8d), duc to the increased expression of β₃-AR. The *FOXC2* transgene possibly actuates other proteins further down the signal transduction pathway finally leading to the induction of UCP1 expression. In addition, the levels of C/EBPα and PPARγ mRNAs in transgenic WAT reaches the ones in witd-type BAT (Fig. 10) and both of these transcription factors are known to induce UCP1 expression (Digby *et al*., 1998: Yubero *et al*., 1994). C/EBPα activates several adipocyte-specific genes and also genes involved in insulin action (Fig. 15), hence C/EBPα (-/-) cells show a complete absence of insulin-stimulited glucose transport, secondary to reduced gene expression and tyrosine phosphorylation for the insulin receptor and IRS-I (Wu *et al*., 19996). The WAT of *FOXC2* transgenic mice have marked elevation of the mRNA levels for both C/EBPα and PPARγ, these transcription factors may in turn be responsible for upregulation of mRNA levels for aP2, LPL. UCP1, GLUT4, insulin receptor, and IRS-1 (Fig. 14)

It is interesting to note that the white adipocytes of *FOXC2* transgenic mice have not just converted into brown adipocytes, in the meaning of mRNA expression, as they have for example higher levels of certain mRNAs (i.e. β₂-AR, insulin receptor, IRS-t, and IRS-2) than seen in any type of wild-type adipose tissue.

In the mice studied here, *FOXC2* transgene expression was under the control of the aP2 promoter, which only functions in adipocytes and not in stem cells and probably neither in intraconveritble cells discussed above (Ailhaud *et al*., 1992). Considering the fact that aP2 is a late marker (Fig. 2) it is quite surprising that we obtain such a dramatic change in the characteristics of white adipocytes. Currently, it is not known if adipocyte dedifferentiation occurs *in vivo*, whereas it has been demonstrated *in vitro* that this process occurs and is induced by TNFα in human adipocytes (Petruschke and Hauner, 1993). Another interpretation for the occurrence of small multilocular cells in WAT of our transgenic mice could then be a dedifferentiation of originally white adipocytes followed by a conversion into the type of adipocytes observed in the *FOXC2* transgenic mice.

The interscapular brown fat of *FOXC2* transgenic mice weighed ~7.5 times as much as wild-type brown fat (Fig. 8a). This extreme hypertrophy might be explained by the increased expression of β₃- and β₂-AR mRNA seen in BAT of *FOXC2* transgenic mice (Fig. 9). Chronic treatment with β₃-AR agonists increases body temperature and energy expenditure and it causes hypertrophy of the interscapular BAT, with several fold increases in the content of UCP1 and cytochrome oxidase (Himms-Hagen *et al*., 1994). The morphology of transgenic interscapular BAT is somewhat changed having larger fat droplets than wild-type BAT (Fig. 7a & b). This is not a feature of chronic β₃-AR agonist treatment, but there is a possibility that the upregulation of markers involved in insulin action (Fig. 9) and elevated levels of adipsin promotes the increased storage of triglycerides in brown adipocytes of transgenic mice. It has also been noticed before that elevated levels of UCP2 mRNA can be coupled to this phenotype (Enerback *et al*., 1997: Kozak *el al*., 1991).

Dysfunctional BAT seen in the ADDI/nSREBP-lc transgene (Shimomura *et al*., 1998) and genetically ablated BAT in the UCP1-DTA transgene (UCP1 promoter - diphtheria toxin A chain) (Lowell *et al*., 1993) leads to insulin resistance. Transgenic mice overexpressing ADD1/nSREBP-1c displays several features quiet opposite the ones of the *FOXC2* transgene, including insulin resistance and NIDDM. *FOXC2* transgenic mice displays a somewhat opposite change of expression pattern compared with that of ADD1/nSREBP-1c transgenic mice, there the mRNAs encoding PPARγ. C/EBPα, aP2, UCP I , adipsin, InsR, IRS-1, IRS-2, and GLUT4 all are downregulated. In our transgene all of this mRNAs are instead upregulated (Fig. 9). However, intriguingly our transgenic mice actually have raised levels of ADD1/SREBP1 mRNA in WAT (Fig. 9) somewhat mimicking the ADD1/nSREBP-1c transgene in that regard.

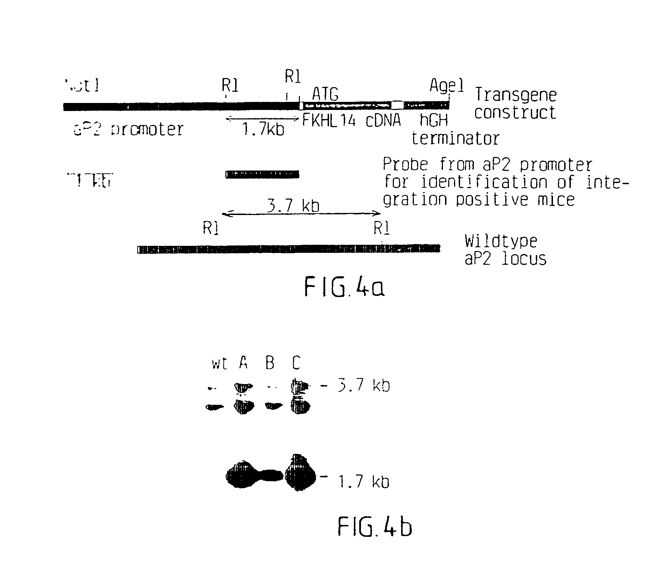

*FOXC2* might be an important participant in the regulation of leptin expression, based on the fact that leptin mRNA levels are down-regulated in *FOXC2* transgenic mice (most prominent in BAT; Fig. 9), and the ten-fold decrease of Ob promoter activity seen in cell culture experiments then cotransfected with *FOXC2* expression plasmid (Fig. 4). In addition, leptin expression is inhibited by P3 -adrenergic stimuli (Mantzoros *et al*., 1996), which is presumed to be high in *FOXC2* transgenic mice due to the up-regulation of β₃-AR mRNA levels.

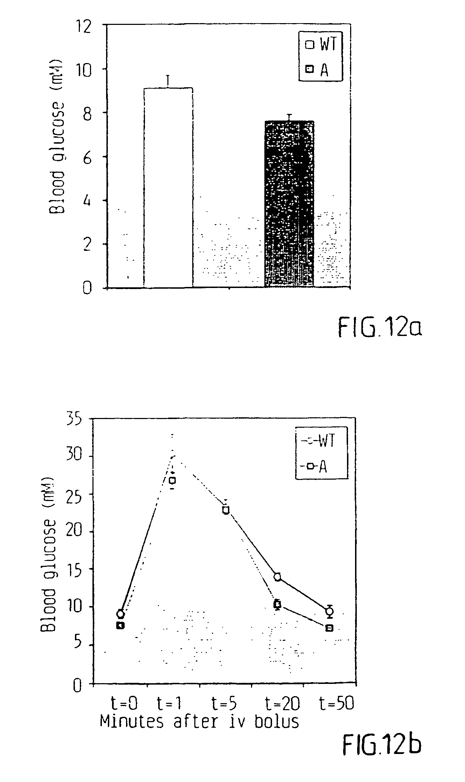

The amount of food consumed by transgenic mice compared to that of wild-type did not differ (Fig. 8d), and no significant difference in body weight has been observed, predicting that the difference observed in total body lipid content must be compensated with an increased anabolism in *FOXC2* transgenic mice. The *FOXC2* transgenic mice most probably also are protected against developing diet-induced obesity, taking in consideration the observed insulin sensitivity (Fig. 13), the lower blood glucose levels and more efficient glucose elimination (Fig. 12) observed in our transgenic mice. Insulin sensitivity and/or resistance to diet-induced obesity have been observed for several other transgenic mouse models: targeted disruption of the RIIβ subunit of protein kinase A results in lean mice resistant to diet-induced obesity (Cummings *et al*., 1996), mice lacking the protein tyrosine phosphatase-1B gene (PTP- 1B) are insulin sensitive and resistant to obesity (Elchebly *et al*., 1999), aP2-UCP1 transgenic mice are prevented against genetic obesity (Kopecky *et al*., 1995), and transgenic mice overexpressing the β₁-AR in adipose tissue are resistant to obesity (Soloveva *et al*., 1997). Moreover, β₃-AR agonists have been found to have anti-diabetic effects in animal models of obesity and NIDDM; chronic dosing can improve glucose tolerance, increase insulin sensitivity and reduce fasting blood glucose levels (Cawthorne *et al*., 1992). *FOXC2* is the only adipocyte specific gene that, directly or indirectly, regulates triglyceride metabolism, adrenergic regulation and insulin action in adipocytes. Actually, the *FKHL14*, is to our knowledge the only known gene that, in a concerted action, can counteract most, if not all, of the symptoms associated with obesity: hypertriglyceridemia, insulin resistance and most likely the associated clinical syndrome of NIDDM.

The apparent *FOXC2* transgene dose responsive effect observed in WAT for the induction of UCP1, β₃-AR, and adipsin, may indicate a direct interaction for *FOXC2* with the promoters of these genes. A schematic view of the hypothetical action of *FOXC2* in adipocytes is shown in Fig. 14.

Proper activation of *FOXC2* by drugs may decrease fat stores, while preserving skeletal muscle mass, by preventing fat assimilation during digestion and by increasing WAT lipolysis, BAT thermogenesis, and insulin action. Such drugs may thus prove useful in treating obesity and NIDDM as well as associated diseases. It is thus foreseen that an effective amount of a polypeptide encoded by the human *FOXC2* gene, could be useful in methods for the treatment of medical conditions related to obesity.

Disclosed is a construct, or more specifically a gene construct or recombinant construct, comprising a human *FOXC2* nucleotide sequence operably linked to an element selected from the group consisting of promoters, response elements, enhancer elements and mixtures thereof. The term "operably linked" as used herein means functionally fusing an element with a structural gene in the proper frame to express the structural gene under control of the element.

Preferably, the said element is a promoter, in particular an adipose-specific promoter such as the adipose-specific promoter of the murine gene encoding adipocyte P2 (Fig. 4), which can be isolated as described by Ross et al. (1990).

Preferably, the said *FOXC2* nucleotide sequence is identical or substantially similar with SEQ ID NO: I of the Sequence Listing. However, the *FOXC2* nucleotide sequence is not to be limited strictly to the sequence shown as SEQ ID NO: 1. It also encompasses nucleotide sequences carrying modifications like substitutions, small deletions, insertions or inversions, which nevertheless encode polypeptides having substantially the biochemical activity of the FOXC2 polypeptide.

Consequently, the said human *FOXC2* nucleotide sequence is selected from:
(a) the nucleotide sequence shown as SEQ ID NO: 1;
(b) nucleotide sequences capable of hybridizing, under stringent hybridization conditions, to a nucleotide sequence complementary to the polypeptide coding region of a nucleotide sequence as defined in (a) and which codes for a biologically active FOXC2 polypeptide, or a functionally equivalent modified form thereof;
(c) nucleic acid sequence which are degenerate as a result of the genetic code to a nucleotide sequence as defined in (a) or (b) and which codes for a biologically active FOXC2 polypeptide, or a functionally equivalent modified form thereof; and
(d) nucleotide sequences which are at least 90% homologous, preferably at least 95% homologous, with the nucleotide sequence shown as SEQ ID NO: 1 in the Sequence Listing.

The term "stringent hybridization conditions" is known in the art from standard protocols (e.g. Ausubel et al) and could be understood as e.g. hybridization to filter-bound DNA in 0.5 M NaHPO₄, 7% sodium dodecyl sulfate (SDS), 1 mM EDTA at +65°C, and washing in 0.1xSSC / 0.1% SDS at +68°C.

Disclosed is a transgenic non-human mammalian animal whose genome comprises a gene construct as defined above, said animal being capable of expressing the human *FOXC2* gene in its adipose tissue.

By "transgenic animal" is meant a non-human mammalian animal that includes a nucleic acid sequence which is inserted into a cell and becomes a part of the genome of the animal that develops from that cell. Such a transgene may be partly or entirely heterologous to the transgenic animal. Other transgenic mammals, including transgenic rodents (for example, hamsters, guinea pigs, rabbits, and rats), and transgenic pigs, cattle, sheep, and goats may be constructed by standard techniques.

One means available for producing a transgenic animal, with a mouse as an example, is as follows: Female mice are mated, and the resulting fertilized eggs are dissected out of their oviducts. The eggs are stored in an appropriate medium. DNA or cDNA encoding the *FOXC2* gene is purified from a vector by methods well known in the art. Tissue specific regulatory elements, such as the adipocyte-specific aP2 promoter discussed above, may be fused with the coding region to permit tissue-specific expression of the trans-gene. The DNA, in an appropriately buffered solution, is put into a microinjection needle and the egg to be injected is put in a depression slide. The needle is inserted into the pronucleus of the egg, and the DNA solution is injected. The injected egg is then transferred into the oviduct of a pseudopregnant mouse, where it proceeds to the uterus, implants, and develops to term.

A transgenic mouse could preferably be derived from a genetically obese mouse. Genetically obese mice, such as ob/ob or db/db mice, are well known in the art.

Disclosed is an isolated cell line derived from the transgenic non-human mammalian animal, as well as a method for producing a transgenic non-human mammalian animal overexpressing the human *FOXC2* gene, said method comprising chromosomally incorporating a gene construct comprising the human *FOXC2* gene, together with suitable regulatory sequences, into the genome of said non-human mammalian animal.

Disclosed is also a method for studying the biological activity of a polypeptide encoded by the human *FOXC2* gene, said method comprising the steps (i) producing a transgenic non-human mammalian animal overexpressing the human *FOXC2* gene; and (ii) comparing the phenotype of the said transgenic non-human mammalian animal with a wild-type animal of the same species.

The invention provides biological screening assays for the identification of compounds that could be useful for the treatment of medical conditions related to obesity, or alternatively, to malnutrition. A "medical condition related to obesity" includes e.g. obesity, NIDDM, hypertension and hyperlipidemia. The said "medical condition related to malnutrition" includes e.g. anorexia, ineffective metabolism, and cancer.

Consequently, the invention provides a method for identifying a compound useful for the treatment of a medical condition related to obesity, said method comprising the steps (i) contacting a test compound with the human *FOXC2* gene; and (ii) determining whether said test compound activates the expression of the human *FOXC2* gene, such activation being indicative for a compound useful for the treatment of a medical condition related to obesity.

Disclosed is also a method of screening for a compound useful for the treatment of a medical condition related to obesity, said method comprising exposing a non-human mammalian animal to a test compound, and determining the activity of said human *FOXC2* gene in said non-human mammalian animal, wherein an increase in said gene activity as compared to an untreated non-human mammalian animal being indicative of a compound useful for the treatment of a medical condition related to obesity.

The said non-human mammalian animal is preferably a mouse, for example an obese mouse. Mice can be rendered obese by administration of a high-fat diet. Alternatively, the mouse can be a genetically obese mouse, such as an ob/ob or db/db mouse.

In the methods described above, the activity of the human *FOXC2* gene in said non-human mammalian animal, can advantageously be compared to the activity of the said human *FOXC2* gene in a transgenic non-human mammalian animal expressing or overexpressing the said human *FOXC2* gene.

In an alternative method for identifying a compound useful for the treatment of a medical condition related to obesity, the method comprises the steps (i) contacting a test compound with a polypeptide encoded by the human *FOXC2* gene; and (ii) determining whether said test compound stimulates the biological activities of the said polypeptide, such stimulation being indicative for a compound useful for the treatment of a medical condition related to obesity. The term "biological activities", as used in this context, means e.g. enhancing the DNA-protein interaction between the FOXC2 polypeptide and target sequences in promoters of target genes. disclosed is a method for identifying a compound useful for the treatment of a medical condition related to malnutrition, said method comprising the steps (i) contacting a test compound with the human *FOXC2* gene; and (ii) determining whether said test compound decreases or inhibits expression of the *FOXC2* gene, such decrease or inhibition being indicative for a compound useful for the treatment of a medical condition related to malnutrition.

Disclosed is also a method of screening for a compound useful for the treatment of a medical condition related to malnutrition, said method comprising exposing a non-human mammalian animal, preferably a mouse, such as an obese mouse, to a test compound, and determining the activity of said human *FOXC2* gene in said non-human mammalian animal, wherein a decrease in said gene activity as compared to an untreated non-human mammalian animal being indicative of a compound useful for the treatment of a medical condition related to malnutrition.

In an alternative method for identifying a compound useful for the treatment of a medical condition related to malnutrition, the method comprises the steps (i) contacting a test compound with a polypeptide encoded by the human *FOXC2* gene; and (ii) determining whether said test compound decreases or inhibits the biological activities of the said polypeptide, such decrease or inhibition being indicative for a compound useful for the treatment of a medical condition related to malnutrition.

In the above discussed methods for identifying compounds decreasing or stimulating *FOXC2* biological activity, other genes encoding forkhead proteins can conveniently be used for comparison (counter-screening) in order to determine the specificity of the test compound. Such forkhead genes, e.g. freac-3 and freac-4, are disclosed e.g. by Pierrou et al. (1994) EMBO Journal 13, 5002-5012.

The skilled person will appreciate that other polypeptides, interacting in the same biological pathways as the FOXC2 polypeptide, will be useful in screening methods for the identification of agents, which agents are useful for the treatment of medical conditions related to obesity or malnutrition. Such alternative target polypeptides could be identified e.g. by a yeast two-hybrid assay (cf. Example 11), or by microarray technology (cf. Example 14). Consequently, disclosed is a method for identifying an agent useful for the treatment of a medical condition related to obesity, said method comprising the steps:
(i) identifying a target polypeptide interacting with the FOXC2 polypeptide;
(ii) contacting a candidate agent with the said target polypeptide; and
(iii) determining whether said candidate agent stimulates the biological activities of the FOXC2 polypeptide, such stimulation being indicative for a compound useful for the treatment of a medical condition related to obesity.

The phrase "determining whether said candidate agent stimulates the biological activities of the FOXC2 polypeptide" is intended to comprise, for instance, determining whether the candidate agent is capable of increasing expression of the FOXC2 polypeptide, or whether the candidate agent is capable of otherwise modulating the biological activities of the FOXC2 polypeptide. The said biological activities of the FOXC2 polypeptide are discussed in detail in the Examples below, and comprises e.g. a reduction of total lipid content in test animals.

For therapeutic uses, the compositions or agents identified using the methods disclosed herein may be administered systemically, for example, formulated in a pharmaceutically acceptable buffer such as physiological saline. Preferable routes of administration include, for example, oral, subcutaneous, intravenous, intraperitoneally, intramuscular, or intradermal injections, which provide continuous, sustained levels of the drug in the patient. Treatment of human patients or other animals will be carried out using a therapeutically effective amount of an identified compound in a physiologically acceptable carrier. Suitable carriers and their formulation are described, for example, in Remington's Pharmaceutical Sciences by E. W. Martin. The amount of the active compound to be administered varies depending upon the manner of administration, the age and body weight of the patient, and with the type of disease and extensiveness of the disease. Generally, amounts will be in the range of those used for other agents used in the treatment of obesity and diabetes.

The human *FOXC2* gene could be used in gene therapy of medical conditions related to obesity. Disclosed is thus a method of treating obesity in a human, comprising (i) administering to the said human a vector comprising a human *FOXC2* DNA sequence operably linked to a promoter; and
(ii) allowing the said human to express a therapeutically effective amount of a polypeptide encoded by said human *FOXC2* gene. A gene delivery system including said vector, comprising a human *FOXC2* DNA sequence operably linked to a promoter, is also disclosed.

The *FOXC2* gene should be operably linked to at least one element which allows for expression of the gene when introduced into the host cell environment. These sequences include promoters, response elements, and enhancer elements. Preferred is the adipose-specific promoter/enhancer of the murine gene encoding adipocyte P2

The heterologous gene may be delivered to the organism using a vector or other delivery vehicle. DNA delivery vehicles can include viral vectors such as adenoviruses, adeno-associated viruses, and retroviral vectors. See, for example: Chu et al. (1994) Gene Ther 1: 292-299; Couture et al. (1994) Hum Gene Ther 5:667-677; and Eiverhand et al. (1995) Gene Ther 2: 336-343. Non-viral vectors which are also suitable include DNA-lipid complexes, for example liposome-mediated or ligand/poly-L-Lysine conjugates, such as asialoglyco-protein-mediated delivery systems. See, for example: Feigner et al. (1994) J. Biol. Chem, 269: 2550-2561; Derossi et al. (1995) Restor. Neurol. Neuros. 8: 7-10; and Abcallah et al. (1995) Biol. Cell 85: 1-7.

If a vector is chosen as the delivery vehicle for the gene, it may be any vector which allows expression of the gene in the host cells. It is preferable if the vector also is one that is capable of integrating into the host genome, so that the gene can be expressed permanently. Ad (adenovirus) vectors have been exploited for the delivery of foreign genes to cells for a number of reasons, including the fact that Ad vectors have been shown to be highly effective for the transfer of genes into a wide variety of tissues *in vivo* and the fact that Ad infects both dividing and non-dividing cells. The vector is administered to the host, generally by intravenous injection. Suitable titers will depend on a number of factors, such as the particular vector chosen, the host, strength of promoter used and the severity of the disease being treated.

Alternatively, it is contemplated that in some human disease states, preventing the expression of, or decreasing the activity of, the human FOXC2 gene will be useful in treating disease states. It is contemplated that antisense therapy or gene therapy could be applied to negatively regulate the expression of the human FOXC2 gene. Antisense nucleic acids (preferably 10 to 20 base-pair oligonucleotides) capable of specifically binding to FOXC2 expression control sequences or FOXC2 RNA are introduced into cells (e.g. by a viral vector or colloidal dispersion system such as a liposome). The antisense nucleic acid binds to the FOXC2 target nucleotide sequence in the cell and prevents transcription and/or translation of the target sequence. Phospborothioate and methylphosphonate antisense oligonucleotides are specifically contemplated. The antisense oligonucleotides may be further modified by poly-L-lysine, transferrin polylysine, or cholesterol moieties at their 5'-end. Suppression of FOXC2 expression at either the transcriptional or translational level is useful to generate cellular or animal models for diseases/conditions characterized by aberrant FOXC2 expression.

Throughout this description the terms "standard protocols" and "standard procedures", when used in the context of molecular biology techniques, are to be understood as protocols and procedures found in an ordinary laboratory manual such as: Current Protocols in Molecular Biology, editors F. Ausubel et al., John Wiley and Sons, Inc. 1994, or Sambrook, J., Fritsch, E.F. and Maniatis, T., Molecular Cloning: A laboratory manual, 2nd Ed., Cold Spring Harbor Laboratory Press, Cold Spring Harbor, NY 1989.

### EXPERIMENTAL METHODS

### Cloning and DNA construct

A human adipose tissue λgt11 cDNA library (Clontech) was screened with a probe mixture corresponding to the conserved fork-head domain derived from: FOXC1, FOXD1, FOXL1, and FOXA1. Hybridization was carried out at low stringency, *i.e.* 6xSSC at +60°C, post-hybridization washes at 0.5xSSC at +60°C. One of the positive recombinants harboring a 2.1 kb insert was subcloned and sequenced. A 5.4-kb *Eco*RV*-Sma*I fragment was excised from pBluescript II SK(+) vector containing the 5.4-kb promoter/enhancer of the mouse aP2 gene and ligated into the *Eco*RV-*Spe*I blunt site of pBluescript II SK(+) vector containing the 2.1 kb FOXC2 cDNA. A 7.6 kb *Xho*I blunt fragment containing the aP2 promoter/enhancer followed by the FOXC2 cDNA was excised from the above plasmid and ligated into the *Ecu*RV site of the pCB6+ vector, which .contains a polyadenylation signal from the human growth hormone gene. After these procedures the resulting 8.2-kb fragment, harboring the aP2-FOXC2 construct with polyadenylation signal, was flanked by the unique sites *Not*I and *Age*I. The plasmid was sequenced over ligation sites.

### Transgenic mice

Construct DNA (aP2-FOXC2), purified using Qiagen kit, according to manufacturer's instructions, was injected into the male pronucleus of (C57BL6 x CBA) F, zygotes, cultured over night and transferred to pseudopregnant females. Tg founder lines were back-crossed to C57BL6/J for four generations. Mice were feed a standard chow with 4% fat content. In experiments with high fat diet mice were fed either a chow with 58% fat or a control diet with 11.4% fat (on a caloric basis; Research Diets) for 7 weeks. High fat chow has a total energy content of 23.4KJ/g, control diet 12.6KJ/g.

### Histology

Tissues were fixed over night in 4% paraformaldehyde in PBS at +4°C, dehydrated, embedded in paraffin, sectioned (6-8 µm) and stained with haematoxylin and eosin.

### Serum and lipid analysis

Plasma insulin was determined radioimmunochemically with the use of a guinea pig anti-rat insulin antibody, ¹²⁵I-labeled porcine insulin as tracer and rat insulin as standard (Linco). Free and bound radioactivity was separated by use of an anti-IgG (goat anti-guinea pig) antibody (Linco). The sensitivity of the assay is 17 pmol/l and the coefficiency of variation is less than 3% at both low and high levels. Plasma glucose was determined with the glucose oxidase method and FFA was measured photometrically. Plasma glucagon was determined radioimmunochemically with the use of a guinea pig antiglucagon antibody specific for pancreatic glucagon, ¹²⁵I-labelled-glucagon as tracer, and glucagon standard (Linco). Free and bound radioactivity was separated by use of an anti-IgG (goat anti-guinea pig) antibody (Linco). The sensitivity of the assay is 7.5 pg/ml and the coefficient of variation is less than 9%. Blood levels of serum cholesterol and triglycerides were determined by fully enzymatic techniques 39,40. Total body lipid was assessed using alcoholic hydroxide digestion with saponification of all lipids, neutralization, followed by enzymatic determination of glycerol.

### Intravenous glucose tolerance test

The mice were anesthetized with an intraperitoneal injection of midazolam 0.4 mg/mouse (Hoffman-La-Roche) and a combination of fluanison (0.9 mg/mouse) and fentanyl 0.02 mg/mouse (Janssen). Thereafter, a blood sample was taken from the retrobulbar, intraorbital, capillary plexus in heparinized tubes, and D-glucose 1g/kg (British Drug Houses) was injected rapidly intravenously. New blood samples were taken after 1. 5, 20, and 50 minutes. Following immediate centrifugation at +4°C, plasma was separated and stored at -20°C or until analysis.

### Northern blot

cDNA probes for mouse FoxC2, aP2, ADD-1/SREBP1, coxII, adipsin, β₁₋₃-AR, GLUT4, IR, IRS1, and IRS2 were prepared by RT-PCR by use of first-strand cDNA from mouse epididymal fat poly(A)⁺ RNA. The PCR primers used to generate these probes were as follows:

cDNA probes were radiolabeled with [α-³²P]dCTP (3000 Ci/mmole) by the random labeling method. Total RNA from mice in each group was pooled, and aliquots of 12 µg were separated on an agarose gel. The filters were hybridized with ³²P-labeled probe (10⁶ cpm/ml) for 1h at 62°C with QuikHyb solution (Stratagene) and washed with 0.1% SDS/0.1 x SSC at +62°C for 3x20 min.

### Transfections and reporter gene analysis

Non-confluent cultures of 3T3-L1 adipocytes were transfected with a CAT reporter (pCAT) driven by the human RIα proximal promoters upstream of the alternatively spliced 1a and 1b leader exons (nucleotides 1509 to 2470 GenBank # Y07641). To control transfection efficiency a pGL3control (Promega) luciferase-encoding vector was used. In cmransfections a *FOXC2* expression vector or vector void of insert was used. Transtcctions were carried out using lipofectamine (Gibco), followed by CAT and luciferase assays.

### PKA immunoblotting and kinase activity

WAT and BAT were treated by a Polytron tissue homogenizer (3 x 15 s) and sonicated, on ice, in a buffer containing 10 mM potassium phosphate, pH 6.8, 150 mM sodium chloride, I mM EDTA, 10 mM CHAPS and protease inhibitors, and centrifuged (15.000 x g) to remove insoluble material. Protein concentrations were determined by Bradford assays (BioRad). For immunoblotting, 30 µg of protein was separated by 10% SDS-PAGE. transferred to PVDF membranes and incubated with anti-RIα and anti-RIIβ mAb. Primary antibodies were detected by HRP-conjugated anti-mouse IgG (Transduction Laboratoriea. 1:5000) and ECL (Amersham). PKA activity was measured using Kemptide (Leu-Arg-Arg-Ala-Ser-Leu-Gly) as substrate in the absence or presence of varying concentrations of cAMP. The low levels of activity not inhibited by PKI (2 µM) were subtracted to determine PKA-specific activity.

### EXAMPLES OF THE INVENTION

Additional features of the invention will be apparent from the following Examples. Examples 1 to 10 are actual, while the remaining Examples are prophetic.

### EXAMPLE 1: Isolation of FOXC2 from human abdominal fat tissue

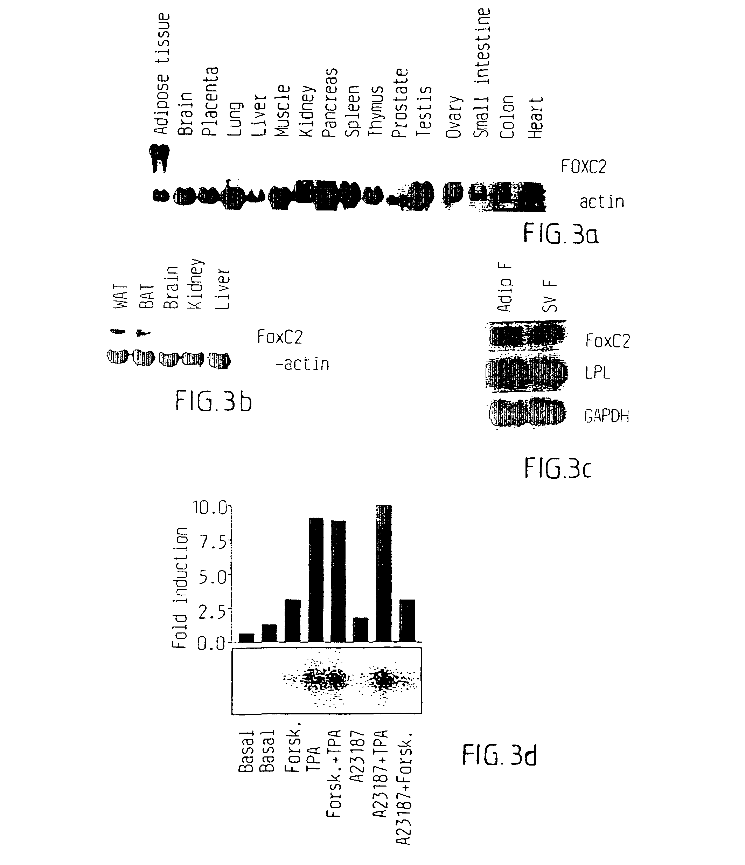

To identify presumptive winged helix genes that are expressed in adipose tissue we screened a Human Fat Cell 5'-STRETCH plus cDNA Library (Clontech) using a low stringency hybridization strategy with a mix of cDNA probes corresponding to DNA-binding domains from different winged helix proteins, to cover the variation between different family members. By this technique, we were able to identify three different forkhead genes, termed *FKHL5, FKHL14*, and *FKHL18, FKHL5* was known to be expressed exclusively in lung and placenta (Pierrou *et al*., 1994) and expression in adipose tissue could not be detected as judged by Northern blot analysis (data not shown). *FKHL18* was identified as a novel member of the forkhead family (Cederberg *et al*., 1997) having a very high homology with the forkhead motif of the mouse gene *fkh-3* (Kaestner *et al*., 1993). Sequence from outside the forkhead motif have not yet been published for *fkh-3* but with regard to the discrepancy of the expression patterns, with *FKHL18* being expressed in arterial vessel wall (aorta) and to a lower extent in kidney and *fkh-3* being expressed in a wide variety of tissues, they are presumed to constitute two different genes. We were not able to detect any expression of *FKHL18* in adipose tissue (data not shown). The third identified gene, *FKHL14* (hereinafter referred to as *FOXC2:* Miura, N. et al. (1997) Genomics 41, 489-492), was shown to be expressed exclusively in human adult adipose tissue by Northern blot analysis (Fig. 3a). The mouse homologue of *FOXC2* is known as *Mfh1* and is expressed in dynamic patterns in the paraxial mesoderm of the trunk and head, in the mesenchyme and endothelial cells of the branchial arches, and in many other sites in the embryo (Kaestner *et al*., 1996; Miura *el al*., 1993: Winnier *et al*., 1997). It has been demonstrated by gene targeting experiments that *Mfh1* plays a crucial role during embryonic development. Homozygous null mutants died pre- and perinatally with multiple skeletal and cardiovascular defects, including defects in the neurocranium and vertebral column, interruptions or coarctations of the aortic arch, and ventricular septal defects (Iida *et al*., 1997; Winnier *et al*., 1997). Heterozygous mice were indistinguishable from wild-type and appeared healthy. The expression of *Mfh1* in adult tissues was restricted to WAT and BAT (Fig. 3b).

### EXAMPLE 2: Physiological studies of FOXC2

In a Northern blot experiment we used RNA from collagenase prepared adipocytes and cells with lower triglyceride content, mostly preadipocytes/adipocytes and cells of stroma/vascular origin, in order to identify the cell type expressing *FoxC2* (Fig. 3c). As can be deduced from Fig. 3c, *FoxC2* expression is highest in the adipocyte fraction with a lower level of expression in stroma/vascular cells, most likely due to the presence of adipocytes with lower triglyceride content - since the adipocyte specific marker LPL (lipoprotein lipase) also is expressed by these cells.

To address the general question of the physiological role/regulation of *FoxC2* we preformed the following experiment: 3T3-L1 adipocytes were stimulated separately and in combination with the cAMP-inducing agent forskolin, the protein kinase C-activator TPA and Ca²⁺-ionophore A23187 to mimic signals elicited down-stream of G-protein coupled receptors. Whereas forskolin alone or in combination with Ca²⁺-ionophore produce a 3-fold increase in steady state levels of *FoxC2* mRNA (Fig. 3d), Ca²⁺-ionophore by itself does not appear to regulate *FoxC2* levels. In contrast, TPA and combinations that include TPA give a strong increase (9 to 10-fold) in *FoxC2* mRNA (Fig. 3d). This suggests that *FowC2* is regulated by intracellular events similar to those elicited by G-coupled hormone receptors.

### EXAMPLE 3: Generation of transgenic mice overexpressing FKHL14

To elucidate whatever function *FOXC2* might have *in vivo* we decided to create transgenic mice overexpressing *FOXC2* in adipose tissue. To achieve overexpression of *FOXC2* in both white and brown adipose tissue, we prepared a transgene construct (Fig. 4a) encoding *FOXC2* driven by a 5.4-kb DNA fragment containing the adipose-specific enhancer/promoter of the gene encoding adipocyte P2 (Ross *et al*., 1990). Integration positive mice were identified using Southern blot analysis (Fig. 4b).

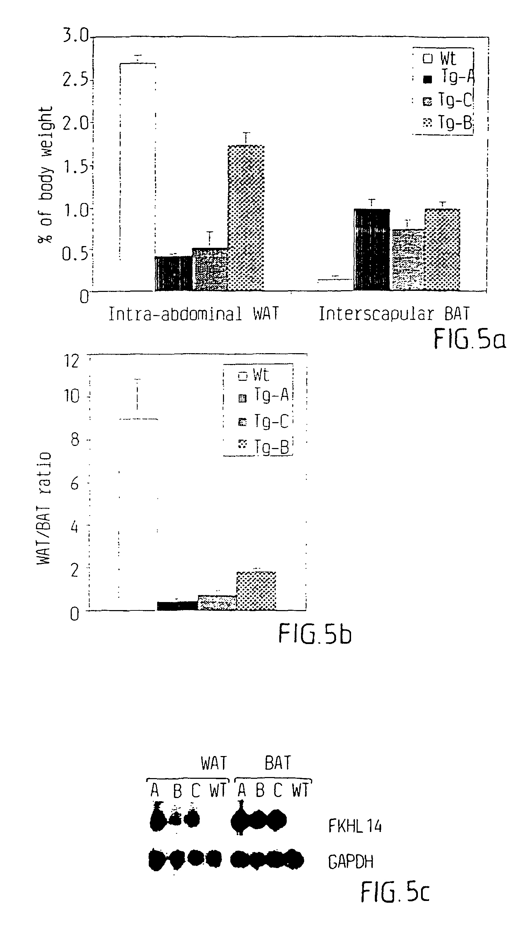

We studied three lines of transgenic mice (A, B, and C) that were derived from independent founders. Transgene expression, analyzed by Northern blot using a *FOXC2* (human specific) probe, was detected at high levels in both WAT and BAT for all three founders. The three different founders had a slight difference in expression levels of the transgene in white fat, founder A having the highest and founder B the lowest, while the expression levels in brown fat were equal (Fig. 5c). All of the transgenic mice used for the study were hemizygous for the transgene and from the second to fourth generation. The transgenic mice appeared normal at external inspection and the transgenic allele was propagated with the expected mendelian distribution. Fed a standard rodent chow diet (4% fat, 18.5% protein, and 55.7% sucrose) weight gain was studied for all founders from 4 weeks up to 6 months of age with no significant difference between nontransgenic and transgenic littermates noticed (data not shown).

We selected three independent founder lines, named tg-A, tg-B and tg-C with tg-A having the highest, tg-B the lowest and tg-C an intermediate level of *FOXC2* expression in WAT. There is a clear dose-response relation between the expression level of the transgene and reduction in the relative weight of the intra-abdominal WAT depot (Fig. 5a). A similar dose-response pattern exists when the weight ratios of intra-abdominal WAT to interscapular BAT are compared (Fig. 5b). The relative weight of the interscapular BAT depot is increased in tg animals but does not exhibit any dose-response association (Fig. 5a), which is in accordance with the fact that there is no difference between tg-A, tg-B and tg-C in expression of the transgene in BAT.

### EXAMPLE 4: WAT of FOXC2 transgenic mice morphologically resembles BAT

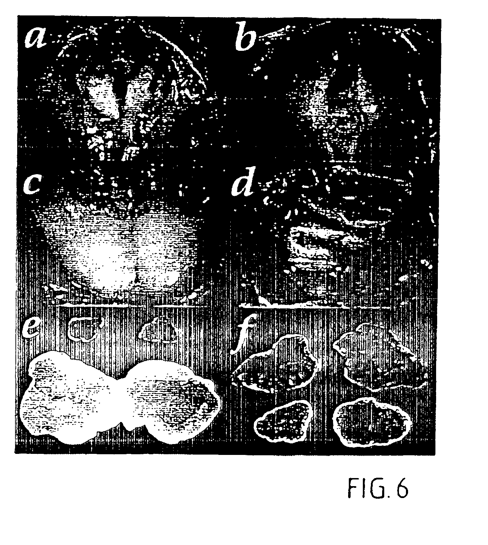

The interscapular brown adipose tissue depot and the intrabdominal white adipose tissue were examined in 5-months-old mice (Fig. 6). Interscapulary, transgenic mice had a greatly enlarged bilobed (dissected apart in the picture) fat pad with the "looks" of brown adipose tissue without the usual coverage of white adipose tissue (Fig. 6b), which normally have to be dissected to uncover the interscapular BAT depot (Fig. 6a). An exposed abdominal view of *FOXC2* transgenic mice revealed an obvious decrease of intrabdominal fat mass and also a distinct change in the appearance towards the brown adipose tissue phenotype (Fig. 6d), when compared to the large, pale lipid-storing intrabdominal fat pad of wild-type mice (Fig. 6c). The adipose tissue change in appearance and mass between transgenic and non-transgenic mice was very striking then the depots had been dissected out (Fig. 6e & f).

Histological analysis of BAT and WAT from 5-months-old mice revealed profound differences between transgenic and non-transgenic tissues. The majority of adipocytes in brown fat of the transgenic mice contained few markedly enlarged fat droplets (Fig. 7b), instead of small multilocular fat droplets typical for BAT (Fig. 7a). Adipocytes in intrabdominal white fat of the transgenic mouse showed heterogeneity of size, all of them having a clearly reduced size (Fig. 7d). This was in contrast to WAT from wild-type mouse, which consisted of large adipocytes of uniform size filled with a large, unilocular lipid-storing vacuole (Fig. 7c).

In a cold adaptation experiment (4°C. 4 hours) tg mice display a clear reduction in size and number of triglyceride droplets in BAT (Fig. 7 e-h). This indicates that the changes in gene expression induced by the transgene will allow a net accumulation of triglyceride at room temperature (22°C) whereas these triglycerides are metabolised at 4°C. In a recent report it has been suggested that, apart from WAT and BAT, aP2 is also expressed in activated macrophages. Measurements of two cytokines IL-12 and IL-18, both implicated in macrophase activation, showed no difference between tg and wt mice (data not shown).

To further examine the change of size of fat depots in transgenic mice, we compared the weights of intrabdominal WAT depots and interscapular BAT depots of 5-months-old wild-type females (n=3) and *FOXC2* transgenic littermates (n=3). In transgenic mice there was a decrease in weight of the intrabdominal WAT depot (-40%) whereas the interscapular BAT depot showed a marked increase (-7.5 times; Fig. 8a). In consequence the ratio between weights of the intrabdominal fat depot and the interscapular brown fat depot was greatly decreased (Fig. 8b). No significant difference in body weight could be detected between transgenic and non-transgenic mice (Fig. 8c), nor could any difference in food consumption be observed then measured during a time period of two months (Fig. 8d).

### EXAMPLE 5: Altered gene expression in FOXC2 transgenic mice

Figure 9 shows an analysis of mRNA steady state levels in white and brown adipose tissue of wild-type mice and the three independent transgenic founders. In wild-type mice, the mRNA for uncoupling protein 1 (UCP1) was detectable only in BAT, but in the transgenic mice expression could be detected in WAT, in a dose responsive manner in proportion to the expression level of transgenic *FKHL14.* The WAT blot and BAT blot had different exposure times, 2 days and 30 minutes respectively. UCP2 seemed to have a tendency to be regulated in the opposite direction as compared with UCP in WAT of transgenic mice. The uncoupling protein expressed in skeletal muscle and BAT, UCP3. showed greatly decreased levels in transgenic BAT. The cytochrome c-oxidase subunit 11 (Coxll), a gene encoded by the mitochondrial genome, was used as a marker for density of mitochondria. The density of mitochondria in WAT and BAT from transgenic animals appeared to be elevated and to a lesser extent reduced, respectively. In wild-type mice, the mRNAs of β₁- and β₃-AR were much lower in WAT than in BAT. The *FOXC2* transgene abolished this discrepancy, raising these mRNAs selectively in WAT so that they became equal, or in the case of β₃-AR even higher, compared to the levels in BAT. The β₂-AR mRNA level was elevated in both WAT and BAT of transgenic mice, reaching levels not seen in nor WAT or BAT of wild-type littermates. White fat depots of transgenic animals exhibited profound increment in four of its mRNAs that are associated with fully differentiated adipocytes, that is. PPARγ2, C/EBPα, aP2, and adipsin. Furthermore PGC-I, a co-activator of PPARγ2, was upregulated in both WAT and BAT. The mRNA for ADDt/SREBP was elevated in WAT for all three founders. The transgenic animals demonstrated a reduction of the amount of leptin mRNA with the most distinct effect in BAT. Steady state levels of LPL mRNA appear to have increased slightly in WAT of transgenic mice. All investigated markers involved in insulin action were upregulated: the insulin receptor (InsR). IRS-I, IRS-2, and insulin-responsive glucose transporter-4 (GLUT4). The upregulation was most evident in WAT. GAPDH was used as a control to verify equal amounts of total RNA in the different lanes.

### EXAMPLE 6: Triglyceride content is altered in FOXC2 transgenic mice

Serum triglyceride content wa:; analyzed for 14-weeks-old wild-type males (n=4) and *FOXC2* transgenic littermates (n=4) fed ad libitum. The transgenic animals exhibited a ~60% reduction in serum triglyceride levels (Fig. 10).

Total body lipid content was assessed using alcoholic potassium hydroxide digestion with saponification of all fats, neutralization, and then enzymatic determination of glycerol (Triglyceride kit, Sigma) as described previously (Salmon and Flatt, 1985). The assay was carried out for 6-months-old wild-type males (n=4) and *FOXC2* transgenic littermates (n=4) fed ad libitum. The total body lipid content was reduced to 10% of the body weight for transgenic mice compared with the normal 30% of body lipid noted for wild-type mice (Fig. 11).

### EXAMPLE 7: Lower blood glucose and more efficient glucose elimination in FOXC2 transgenic mice

Nonfasting blood glucose levels were measured for 10-weeks-old wild-type males and females (n=5+5), and *FOXC2* transgenic littermates (n=5+5) fed ad libitum. The transgenic animals showed a significant 16% reduction in nonfasting blood glucose levels (Fig. 12a).

A glucose tolerance test was performed on 10-weeks-old wild-type males and females (n=5+5) and *FOXC2* transgenic littermates (n=5+5) fed ad libitum. Plasma glucose levels peaked at one minute after intravenous glucose administration, and thereafter plasma glucose levels returned to baseline levels within the 50-minutes study period. Transgenic mice displayed an enhanced glucose elimination, with plasma glucose levels significantly lower than wild-type littermates at 0, 20, and 50 minutes (P<0.05; P<0.001; P<0.05) (Fig. 12b). Data are means of both males and females as no difference between gender was noticed.

### EXAMPLE 8: Increased insulin sensitivity in FOXC2 transgenic mice

Non-fasting plasma insulin levels in *FOXC2* transgenic mice were analyzed using the same group of animals as was used for blood glucose measurements. The concentration of plasma insulin in *FOXC2* transgenic mice before the start of the glucose tolerance test were reduced to -50% of the levels registered for wild-type littermates (Fig. 13a). The rapid intravenous injection of glucose (1 g/kg) raised plasma insulin levels 4-fold in wild-type mice and 10-fold in *FOXC2* transgenic littermates after one minute (Fig. 13c). Thereafter, plasma insulin levels rapidly returned toward baseline values observed before the glucose load, with transgenic mice having significantly lower levels at 20 and 50 minutes (P<0.05; P<0.05) (Fig. 13b). Data are means of both males and females as no difference between gender was noticed.

In mice on a high fat diet, there are significantly lower weight gains in transgenic mice as compared with wt. In females the weight gain is 39% (p<0.02; Fig. 15a) lower as compared with wt females and for males the difference is 21% (p<0.03; Fig. 15b). These findings highlight *FOXC2*, not only as a gene of importance for adipose tissue distribution, morphology and gene cxprcssion profile, but also, more importantly, as a major regulator of general lipid and glucose metabolism including protection against diet induced weight gains.

### EXAMPLE 9: FOXC2 regulates body composition, serum lipids and insulin sensitivity

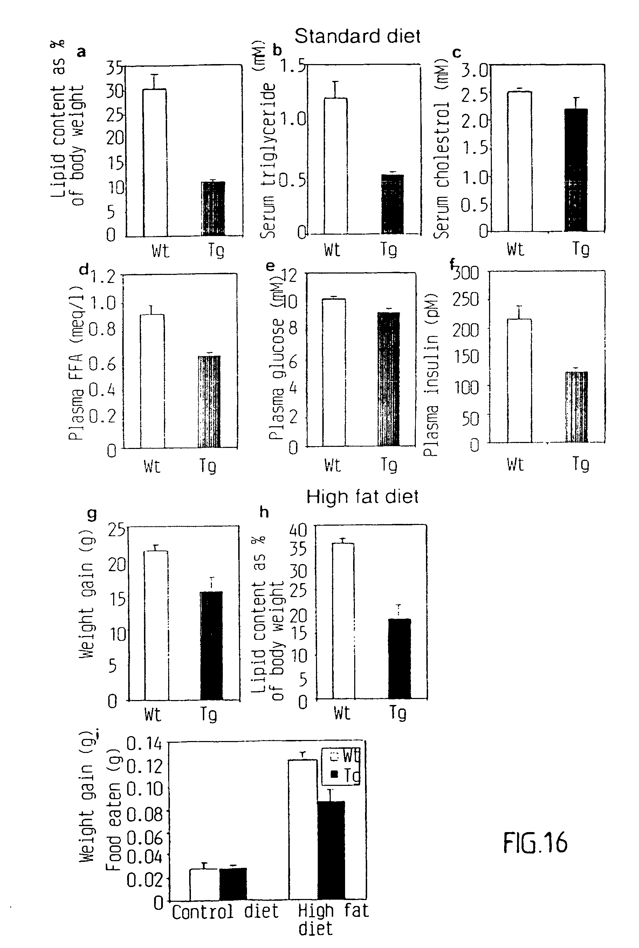

Several physiological tests and measurements of critical metabolites with relevance for obesity, insulin resistance and type 2 diabetes were carried out in order to assess the impact of increased *FOXC2* expression on systemic lipid and glucose metabolism. We assayed total body lipid content on whole carcasses using the alcoholic potassium hydroxide digestion method and found a decrease from 30% total lipids in carcasses from wt mice as compared with only 10% in tg mice (p<0.0004; Fig. 16a). There is also a highly significant reduction in serum triglyceride levels by 57% (p<0.004; Fig. 16b), whereas there is no significant change in serum cttolesterol levels (Fig. 16c). FFA are lowered from 0.92mcq/l to 0,63meq/1 in tg animals (p<0.02; Fig. 16d). In *ad libitum* fed animals we note a decrease in plasma glucose levels by 10% (p<0.01; Fig. 16e) and plasma insulin levels are down by 43% (p<0.001; Fig. 16f). In mice on a high fat diet, the weight gain is 28% lower (p<0.01; Fig. 16g) for tg mice as compared with wt litter mates. Although lipid content in tg and wt mice are increased after a high fat diet tg mice still resist lipid accumulation to the same degree as is the case for wt litter mates, a 49% decrease in tg mice as compared with wt (p<0.0001; Fig. 16h). On a control diet (4.8% fat) tg and wt mice display no signs of differences in metabolic efficiency as judged by calculating the ratio between weight gain and weight of food eaten (Fig. 16i). When fed a high fat diet (35.9% fat) tg mice appear less efficient in their metabolism since they need to eat more for each gram of increase in body weight (a reduction by 30% p<0.01; Fig. 16i). These findings are interesting since they suggest that metabolic efficiency, in *FOXC2* tg can be regulated in response to food composition. There are no significant differences in serum glucagon levels, body weight or food consumption (on standard diet) between wt and tg animals (not shown) nor have we observed any significant gender differences.

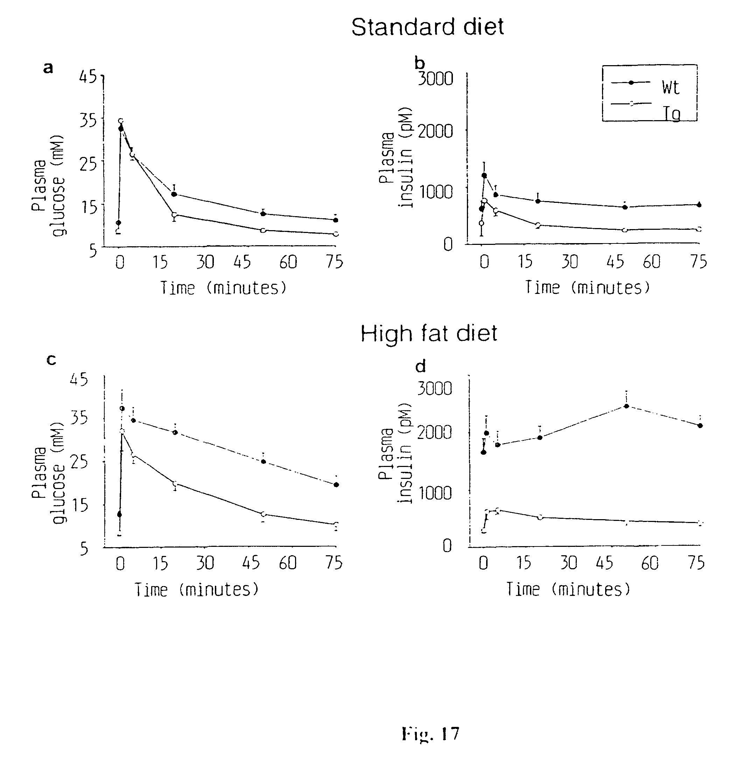

In an intravenous glucose tolerance test, plasma glucose was assayed at 0, 1, 5, 20, 50 and 75 minutes after glucose administration as depicted in Fig. 17a. Plasma glucose levels for tg mice on control diet are significantly lower at 0, 20 and 75 minutes (p<0.05), and at 50 minutes (p<0.02). The insulin curve for the same experiment exhibit lower insulin levels for tg mice (Fig. 17b) at 0 minutes (p<0.02), at 1, 5 and 20 minutes (p<0.05) and at 50 and 75 minutes (p<0.001). When on a high fat diet the difference after an intravenous glucose tolerance test is much more pronounced (Fig. 17c and d). Glucose levels are lower in tg mice as compared with wt at 0, 5, 20, 50 and 75 minutes (p<0,001; Fig. 17c). Insulin levels are dramatically lowered in tg mice at all time points (p<0.001; Fig. 17d). It is quite extraordinary that tg mice on a high fat diet retain a plasma insulin profile almost identical to that observed when on a standard diet while wt mice display almost a 3-fold increase in insulin plasma levels (Fig. 17 b, d). In spite of this wt mice exhibit a clear increase in glucose levels while tg mice show a much more modest increase in glucose values (Fig. 17 a, b). These findings highlight *FOXC2,* not only as a gene of importance for adipose tissue distribution, morphology and gene expression profile, but also, more importantly, as a major regulator of general lipid and glucose metabolism including protection against diet induced insulin resistance. EXAMPLE 10: Increased sensitivity of the β-adrenergic/cAMP/protein kinase A pathway

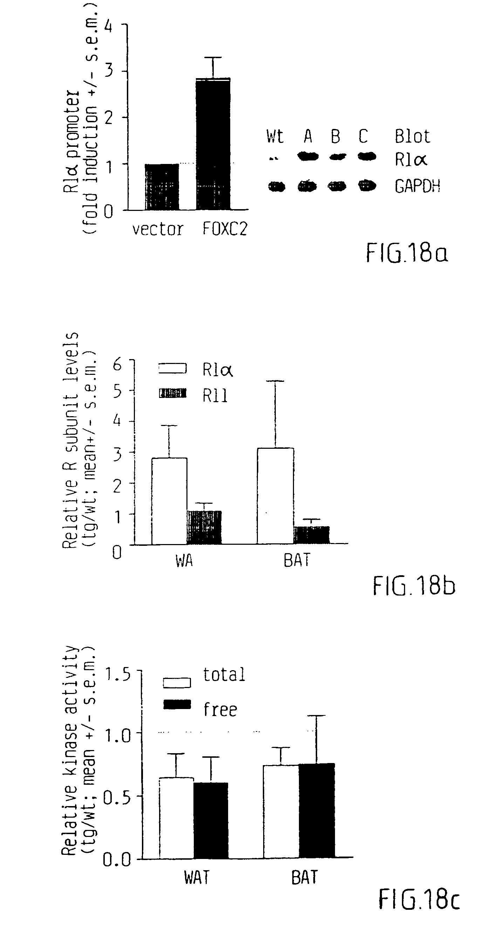

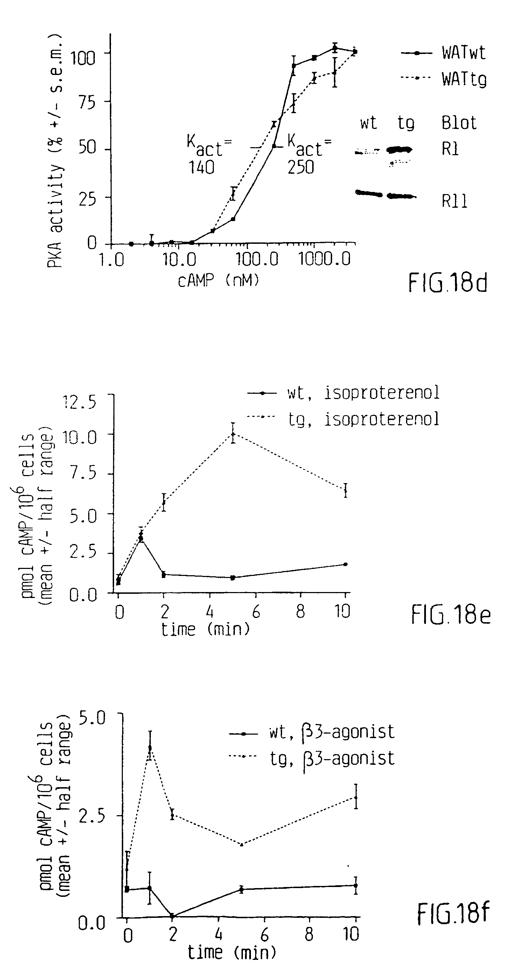

The induction of the cAMP-regulated ucpl in WAT, the hypertrophy of BAT, the decrease of intra-abdominal WAT depots and the up-regulation of mRNAs encoding β-ARs suggest that an increased perturbation and sensitivity in the β-adrenergic/cAMP/protein kinase A (PKA) signaling pathway may contribute to the phenotype of mice over-expressing *FOXC2*. In cotransfection experiments, using 3T3-L1 adipocytes, we show that *FOXC2* increases reporter gene activity of a construct driven by the promoter of the *RI*α gene (encodes the regulatory subunit Iα of PKA; Fig. 18a), whereas no such regulation could be observed for the *RIIβ* promoter (data not shown). Consistent with these findings we also demonstrate increased *RI*α mRNA levels in adipose tissue from tg mice as compared with wt litter mates (Fig. 18a, insert), in a dose dependent manner with regard to transgene expression. This is accompanied by elevated levels of RIα protein and unchanged (WAT) or decreased (BAT) levels of RIIβ protein in tg mice (Fig. 18b). However, basal and total PKA activity is decreased in tg WAT and BAT (Fig. 18c). This may be due to an increased sensitivity to activation by cAMP, dissociation and thereby degradation of PKA holoenzyme. The PKA type I holoenzyme (RIα₂C₂) binds cAMP with higher affinity and activates more easily than the PKA type 2 enzyme (RIIβ₂C₂), normally expressed in WAT and BAT. Indeed, PKA from WAT of *FOXC2* tg mice with elevated protein levels of RIα (Fig. 18d, insert), activates more easily than PKA from WAT of non-tg litter mates (Fig. 19d, K_{act} of 140 and 250 nM, respectively) although protein levels of RIIβ appear unchanged (Fig. 18d, insert). Thus, adipocytes from *FOXC2* tg mice will have a lower threshold for PKA activation by adrenergic stimuli as compared with wild type litter mates due to a PKA isozyme switch. This is in accordance with what has been reported for mice with targeted disruption of the *RIIβ* gene, where an isozyme-switch from PKA type II (RIIβ₂C₂) to PKA type I (RIα₂C₂) with a change in K_{act} from 220 nM to 80 nM in WAT leads to a lean mouse with similar phenotype as the *FOXC2* tg mouse. In addition to the increased sensitivity of the PKA-system in *FOXC2* tg adipose tissue, examination of the β-adrenergic sensitivity of WAT-adipocytes from *FOXC2* tg and wt litter mates revealed distinct differences. Whereas stimulation of wt adipocytes with a non-selective β-agonist leads to a 4 to 5-fold increase in cAMP levels at 1 min and a rapid desensitization of the signal, stimulation of of tg adipocytes leads to a strong (10-fold), prolonged and sustained increase in cAMP levels which is not desensitized at 10 min (Fig. 18e). Furthermore, β₃-agonist stimulation of adipocytes prepared from tg WAT displays a distinct increase (4-fold) in cAMP levels with elevated levels over 10 min (Fig. 19f). In contrast, little or no β₃-agonist response is observed in adipocytes from wt WAT. These observations are in agreement with the strong up-regulation of β₁₋₂-AR and the introduction of β₃-AR (which is virtually absent in wt) in tg WAT and indicate that the sensitivity of β-adrenergic/cAMP/PKA pathway in WAT from *FOXC2* tg mice is increased at several levels.

### EXAMPLE 11: Interaction trap / two-hybrid system

In order to assay for polypeptides interacting with the FOXC2 polypeptide, the interaction trap/yeast two-hybrid (Y2H) library screening method is used. This *in vivo* assay in was first described in Fields & Song (1989) Nature 340, 245-246. A recent method named Sos recuitment system (SRS) has been described by Aroheim & Karin (1997) Mol. Cell. Biol. 17, 3094-3102. The SOS system differs from the original yeast 2-hybrid for at the interaction between bait and prey takes place in the cytoplasm. A kit for the SOS-Y2H is available from Stratagene, La Jolla-CA, USA (Cyto TrapTwo-Hybrid System).

A fusion of an *FOXC2* nucleotide sequence and the human Sos catalytic domain without its C-terminal regulatory domain (Bait) is constructed in an appropriate plasmid (e.g. pSos) using standard subcloning techniques. Similarly, a myristylation sequence fusion library (Prey) is constructed in a second plasmid (e.g. pMyr) from cDNA of potential binding proteins. The Sos fusion construct is verified by sequencing, and tested for its inability to allow growth of cdc25H yeast strain at the restrictive temperature of 37°C and cell toxicity, both of which would prevent a successful two-hybrid analysis. The Myr sequence anchors the Myr/library fusion polypeptides to the plasma membrane and its expression depends on the sugar-containing medium used. Yeast cells are transformed (ca. 5-10 x 10⁶ transformants/mg DNA) with both the Sos and Myr library fusion plasmids according to standard procedures. *In vivo* binding of Sos/(FOXC2) with Myr/library proteins results in a recruitment of the hSos protein to the membrane, thereby activating the Ras-signaling pathway and allowing the cdc25H yeast cells to grow at 37°C. Yeast cells are first plated on a glucose media lacking the amino acids Leu and Ura to select both plasmids. After 4-5 days at 25°C, plates are used for replica plating onto galactose (-Leu, -Ura) plates incubated at 37°C for 3-10 days . Colonies that are growing are first tested for their ability to grow at 37°C depending of the sugar present in the medium. Those clones that show growth in galactose compared to glucose are considered candidates. To test the specificity of the of the library plasmids, plasmid DNA is extracted from candidate clones and used to cotransform cdc25H cells with either the specific bait or a non-relevant bait. Insert DNA is sequenced to verify the presence of an open reading frame fused to the Myr sequence and to determine the identity of the interacting protein.

### EXAMPLE 12: Mobility shift DNA-binding assay

A gel electrophoresis mobility shift assay, according to standard procedures, can rapidly detect specific protein-DNA interactions. Probe DNA (<300 bp) is obtained from synthetic oligonucleotides, restriction endonuclease fragments, or PCR fragments and end-labeled with ³²P. An aliquot of purified FOXC2 (ca. 15 µg) or crude FOXC2 extract (ca. 15 ng) is incubated at constant temperature (in the range 22-37°C) for at least 30 minutes in 10-15 µl of buffer (i.e. TAE or TBE, pH 8.0-8.5) containing radiolabeled probe DNA, nonspecific carrier DNA (ca. 1 µg), BSA (300 µg/ml), and 10% (v/v) glycerol. The reaction mixture is then loaded onto a polyacrylamide gel and run at 30-35 mA until good separation of free probe DNA from protein-DNA complexes occurs. The gel is then dried and bands corresponding to free DNA and protein-DNA complexes are detected by autoradiography.

### EXAMPLE 13: Reporter gene assay to identify modulating compounds

Reporter gene assays are well known as tools to signal transcriptional activity in cells. (For a review of chemiluminescent and bioluminescent reporter gene assays, see Bronstein et al. (1994) Analytical Biochemistry 219, 169-181.) For instance, the photoprotein luciferase provides a useful tool for assaying for modulators of FOXC2 activity. Cells (e.g., CHO cells or COS 7 cells) are transiently co-transfected with both a FOXC2 expression construct and a reporter construct which includes a gene for the luciferase protein downstream from a transcription factor binding site. Agonist binding to FOXC2 results in expression of the luciferase gene. Luciferase activity may be quantitatively measured using e.g. luciferase assay reagents that are commercially available from Promega (Madison, WI). Differences in luminescence in the presence versus the absence of a candidate modulator compound are indicative of modulatory activity.

### EXAMPLE 14: Expressing profiling and target identification using microarrays

To better understand the role of FOXC2 and thus identify new drug targets, the expression patterns of a large number of genes and ESTs can be monitored using GeneChip® expression microarrays (*http:*//*www.affymetrix.com*/*products*/ *app_exp.html*). Microarrays consist of a highly ordered matrix of thousands of different sequences that can be used to measure DNA and RNA variation in applications that include gene expression profiling, comparative genomics and genotyping (for recent reviews, see e.g.: Harrington et al. (2000) *Monitoring gene expression using DNA microarrays.* Curr. Opin. Microbiol. 3(3): 285-291; or Dugan et al. (1999) *Expression profiling using cDNA microarrays.* Nature Genetics supplement 21:10-14).

Briefly. RNAs are extracted from any tissues or cultured cells relevant for the study of FOXC2 and reverse transcribed using a T7-tagged oligo-dT primer to generate doublestranded cDNAs. These cDNAs are then amplified and labeled using In Vitro Transcription (IVT) with T7 RNA polymerase and biotinylated nucleotides. The populations of cRNAs obtained after IVT are purified and fragmented by heat to produce a distribution of RNA fragment sizes from approximately 35 to 200 bases. GeneChip® expression arrays are hybridized with the samples. The arrays are washed and stained. The cartridges are scanned using a confocal scanner and the images are analyzed with the Microarray Suite 4.0 software (Affymetrix).

Using this analysis the role of FOXC2 in specific signaling pathways can be elucidated, and new signaling pathways can be identified. Genes directly or indirectly regulated by FOXC2 can be identified. The analysis will identify genes with previously known function but also novel genes. Gene products functioning in the same pathways as FOXC2 can be considered putative target proteins. Targeting of such proteins would be expected to yield some or all of the desired effects expected from targeting FOXC2 directly.

### REFERENCES

Ailhaud, G.. Grimaldi, P., and Negrel, R. (1992). Cellular and molecular aspects of adipose tissue development. *Annu Rev Nutr* **12,** 207-33.

Arch, J. R., and Wilson, S. (1996). Prospects for beta 3-adrenoceptor agonists in the treatment of obesity and diabetes. *Int J Obes Relat Metab Disord* **20,** 191-9.

Barak. Y., Nelson, M. C., Ong, E. S.. Jones, Y. Z., Ruiz-Lozano, P., Chien, K. R., Koder, A., and Evans, R. M. (1999). PPAR gamma is required for placental, cardiac, and adipose tissue development. *Mol Cell* **4,** 585-95.

Bloom, J. D., Dutia, M. D., Johnson, B. D., Wissner, A., Bums, M. G., Largis, E. E., Dolan, J. A., and Claus, T. H. (1992). Disodium (R,R)-5-[2-[[2-(3-chlorophenyl)-2-hydroxyethyl]-amino] propyl]- 1,3-bcnzodioxole-2.2-dicarboxylate (CL 316,243). A potent beta- adrenergic agonist virtually specific for beta 3 receptors. A promising antidiabetic and antiobesity agent. *J Med Chem* **35,** 3081-4.

Boss, O., Samec, S., Paoloni-Giacobino, A.. Rossier, C., Dulloo, A., Seydoux, J., Muzzin. P.. and Giacobino, J. P (1997). Uncoupling protein-3: a new member of the mitochondrial carrier family with tissue-specific expression. *FEBS Lett* **408,** 39-42.

Brewer, C. B. (1994). Cytomegalovirus plasmid vectors for permanent lines of polarized epithcllal cells. *Methods Cell Biol* **43,** 233-45.

Brown, M. S., and Goldstein, J. L. (1997). The SREBP pathway: regulation of cholesterol metabolism by proteolysis of a membrane-bound transcription factor. *Cell* **89,** 331-40.

Cao. Z., Umek, R. M., and McKnight, S. L. (1991). Regulated expression of three C/EBP isoforms during adipose conversion of 3T3-LI cells. *Genes Dev* **5,** 1538-52.

Cawthorne, M. A.. Sennitt. M. V.. Arch. J. R., and Smith, S. A. (1992). BRL 35135, a potent and selective atypical beta-adrenoceptor agonist. *Am J Clin Nutr* 55, 252S-257S.

Cederberg. A., Bctz, R.. Lagcrcrantz. S., Larsson, C.. Hulander. M., Carlsson, P., and Enerback. S. (1997). Chromosome localization, sequence analysis, and expression pattern identify FKHL 18 as a novel human forkhead gene. *Genomics* **44,** 344-6.

Champigny, O., and Ricquier. D. (1996). Evidence *from in vitro* differentiating cells that adrenoceptor agonists can increase uncoupling protein mRNA level in adipocytes of adult humans: an RT-PCR study. *J Lipid Res* **37,** 1907-14.

Choy, L. N., and Spiegelman. B. M. (1996). Regulation of alternative pathway activation and C3a production by adipose cells. *Obes Res* **4,** 521-32.

Christy. R. J.. Yang. V. W., Ntambi, J. M., Geiman, D. E., Landschulz, W. H., Friedman, A. D., Nakabeppu, Y., Kelly, T. J., and Lane, M. D. (1989). Differentiation-induced gene expression in 3T3-Li prcadipocytes: CCAAT/enhancer binding protein interacts with and activates the promoters of two adipocyte-specific genes. *Genes Dev* **3,** 1323-35.

Chua, S. C., Jr.. Chung, W. K., Wu-Peng, X. S., Zhang, Y., Liu, S. M., Tartaglia, L., and Leibel, R. L. (1996). Phenotypes of mouse diabetes and rat fatty due to mutations in the OB (leptin) receptor. *Science* **271**, 994-6.

Cianflone, K., Maslowska, M., and Sniderman, A. (1995). The acylation stimulating protein-adipsin system. *Int J Obes Relat Metab Disord* **19 Suppl 1,** S34-8.

Coleman, D. L. (1973). Effects of parabiosis of obese with diabetes and normal mice. *Diabetologia* **9,** 294-8.

Cornelius, P.. MacDougald, O. A., and Lane, M. D. (1994). Regulation of adipocyte development. *Annu Rev Nutr* **14,** 99-129.

Cousin, B., Cinti, S., Morroni, M.. Raimbault, S.. Ricquier, D.. Penicaud, L., and Casteilla, L. (1992). Occurrence of brown adipocytes in rat white adipose tissue: molecular and morphological characterization. *J Cell Sci* **103, 931-42.**

Cummings, D. E., Brandon, E. P., Planas, J. V., Motamed. K., Idzerda, R. L., and McKnight, G. S. (1996). Genetically lean mice result from targeted disruption of the RII beta subunit of protein kinase A. *Nature* **382,** 622-6.

De Vos, P., Lefebvre. A. M., Milier, S. G., Guerre-Millo. M., Wong, K., Saladin. R., Hamann, L. G., Staels, B.. Briggs, M. R., and Auwerx, J. (1996). Thiazolidinediones repress ob gene expression in rodents via activation of peroxisome proliferator-activated receptor gamma. *J Clin Invest* **98,** 1004-9.

Digby, J. E., Montague, C. T., Sewter, C. P., Sanders, L., Wilkison, W. O., O'Rahilly, S., and Prins. J. B. (1998). Thiazolidinedione exposure increases the expression of uncoupling protein 1 in cultured human preadipocytes. *Diabetes* **47,** 138-41.

Elchebly, M., Payette, P., Michaliszyn, E., Cromlish, W., Collins, S., Loy, A. L., Normandin, D., Cheng, A., Himms-Hagen, J., Chan, C. C., Ramachandran, C., Gresser, M. J., Tremblay, M. L., and Kennedy, B. P. (1999). Increased insulin sensitivity and obesity resistance in mice lacking the protein tyrosine phosphatase-1B gene. *Science* **283,** 1544-8.

Enerback, S., Jacobsson, A., Simpson, E. M., Guerra, C., Yamashita, H., Harper, M. E., and Kozak, L. P. (1997). Mice lacking mitochondrial uncoupling protein are cold-sensitive but not obese. *Nature* **387,** 90-4.

Enerback, S., Ohlsson, B. G., Samuelsson, L., and Bjursell, G. (1992). Characterization of the human lipoprotein lipase (LPL) promoter: evidence of two cis-regulatory regions, LP-alpha and LP-beta, of importance for the differentiation-linked induction of the LPL gene during adipogenesis. *Mol Cell Biol* **12,** 4622-33.

Fajas, L., Auboeuf, D., Raspe, E.. Schoonjans, K., Lefebvre, A. M., Saladin, R., Najib, J., Laville, M., Fruchart, J. C., Deeb. S., Vidal-Puig, A.. Flier. J., Briggs. M. R., Staels, B., Vidal. H., and Auwerx, J. (1997). The organization, promoter analysis, and expression of the human PPARgamma gene. *J Biol Chern* **272,** 18779-89.

Fajas. L., Schoonjans, K.. Gelman, L., Kirn, J. B., Najib, J., Martin. G., Fruchart. J. C., Briggs, A1., Spiegelman. B. M., and Auwerx, J. (1999). Regulation of peroxisome proliferator-activated receptor gamma expression by adipocyte differentiation and determination factor I/sterol regulatory element binding protein 1: implications for adipocyte differentiation and metabolism. *Mol Cell Biol* **19**, 5495-503.

Fleury, C., Neverova, M., Collins. S., Raimbault, S., Champigny, O., Levi-Meyrueis, C., Bouillaud, F., Seldin, M. F., Surwit, R. S., Ricquier, D., and Warden, C. H. (1997). Uncoupling protein-2: a novel gene linked to obesity and hyperinsulinemia [see comments]. *Nat Genet* **15**, 269-72.

Forman, B. M., Tontonoz, P., Chen, J.. Brun. R. P., Spiegelman, B. M., and Evans, R. M. (1995). 15-Deoxy-delta 12, 14-prostaglandin J2 is a ligand for the adipocyte determination factor PPAR gamma. *Cell* **83,** 803-12.

Furumoto, T. A., Miura, N., Akasaka, T., Mizutani-Koseki, Y., Sudo, H., Fukuda, K., Maekawa, M., Yuasa, S., Fu, Y., Moriya, H., Taniguchi, M., Imai, K.. Dahl, E., Balling, R., Pavlova, M., Gossler, A., and Koseki, H. (1999). Notochord-dependent expression of MFH1 and PAX1 cooperates to maintain the proliferation of sclerotome cells during the vertebral column development. *Dev Biol* **210,** 15-29.

Ghorbani, M., Claus, T. H., and Himms-Hagen, J. (1997). Hypertrophy of brown adipocytes in brown and white adipose tissues and reversal of diet-induced obesity in rats treated with a beta3- adrenoceptor agonist. *Biochem Pharmacol* **54,** 121-31.

Giacobino, J. P. (1995). Beta 3-adrenoceptor: an update. *Eur J Endocrinol* **132,** 377-85.

Granneman, J. G., and Lahners, K. N. (1994). Analysis of human and rodent beta 3-adrenergic receptor messenger ribonucleic acids. *Endocrinology* **135,** 1025-31.

Granneman, J. G.. Lahners, K. N., and Chaudhry, A. (1991). Molecular cloning and expression of the rat beta 3-adrenergic receptor. *Mol Pharmacol* **40,** 895-9.

Himms-Hagen, J. (1989). Brown adipose tissue thermogenesis and obesity. *Prog Lipid Res* **28**, 67-115.

Himms-Hagen, J., Cui, J., Danforth. E., Jr., Taatjes, D. J., Lang. S. S., Waters, B. L., and Claua, T. H. (1994). Effect of CL-316.243. a thermogenic beta 3-agonist, on energy balance and brown and white adipose tissues in rats. *Am J Physiol* **266,** R1371-82.

Hofmann, C., Lorenz, K., Braithwaite, S. S., Colca, J. R., Palazuk, B. J., Hotamisligil, G. S., and Spiegelman, B. M. (1994). Altered gene expression for tumor necrosis factor-alpha and its receptors during drug and dietary modulation of insulin resistance. *Endocrinology* **134, 264-70.**

Holloway, B. R., Howe, R., Rao, B. S., and Stribling, D. (1992). ICI D7114: a novel selective adrenoceptor agonist of brown fat and thermogenesis. *Am J Clin Nutr* **55, 262S-264S.**

Hotamisligil, G. S., Arner, P., Caro, J. F., Atkinson, R. L., and Spiegelman, B. M. (1995). Increased adipose tissue expression of tumor necrosis factor-alpha in human obesity and insulin resistance. *J Clin Invest* **95,** 2409-15.

Hotamisligil, G. S., Shargill, N. S., and Spiegelman, B. M. (1993). Adipose expression of tumor necrosis factor-alpha: direct role in obesity-linked insulin resistance. *Science* **259,** 87-91.

Iida, K., Koseki, H., Kakinuma, H., Kato, N., Mizutani-Koseki, Y., Ohuchi, H., Yoshioka, H., Noji, S., Kawamura, K., Kataoka, Y., Ueno. F., Taniguchi, M., Yoshida, N., Sugiyama, T., and Miura, N. (1997). Essential roles of the winged helix transcription factor MFH-1 in aortic arch patterning and skeletogenesis. *Development* **124,** 4627-38.

Ito, M.. Grujic, D., Abel, E. D., Vidal-Puig, A., Susulic, V. S., Lawitts, J., Harper, M. E., Himms-Hagen, J., Strosberg, A. D., and Lowell, B. B. (1998). Mice expressing human but not murine beta3-adrenergic receptors under the control of human gene regulatory elements. *Diabetes* **47,** 1464-71.

Jezek, P., Orosz, D. E., Modriansky, M., and Garlid, K. D. (1994). Transport of anions and protons by the mitochondrial uncoupling protein and its regulation by nucleotides and fatty acids. A new look at old hypotheses. *J Biol Chem* **269,** 26184-90.

Kaestner. K. H., Bleckmann, S. C., Monaghan, A. P., Schlondorff, J., Mincheva, A., Lichter, P., and Schutz, G. (1996). Clustered arrangement of winged helix genes fkh-6 and MFH-1: possible implications for mesoderm development. *Development* **122,** 1751-8.

Kaestner, K. H., Christy, R. J., and Lane, M. D. (1990). Mouse insulin-responsive glucose transporter gene: characterization of the gene and trans-activation by the CCAAT/enhancer binding protein. *Proc Natl Acad Sci USA* **87,**251-5.

Kaestner, K. H., Lee, K. H., Schlondorff, J.. Hiemisch, H., Monaghan, A. P., and Schutz, G. (1993). Six members of the mouse forkhead gene family are developmentally regulated. *Proc Natl Acad Sci USA* **90,** 7628-31.

Kim, J. B., and Spiegelman, B. M. (1996). ADD1/SREBP1 promotes adipocyte differentiation and gene expression linked to fatty acid metabolism. *Genes Dev* **10**, 1096-107.

Kim, J. B., Wright, H. M., Wright, M., and Spiegelman, B. M. (1998). ADD1/SREBP1 activates PPARgamma through the production of endogenous ligand. *Proc Nail Acad Sci USA* **95,** 4333-7.

Klaus. S. (1997). Functional differentiation of white and brown adipocytes. *Bioessays* **19,** 215-23.

Kopecky. J., Clarke, G.. Enerback, S., Spiegelman, B., and Kozak, L. P. (1995). Expression of the mitochondrial uncoupling protein gene from the aP2 gene promoter prevents genetic obesity. *J Clin Invest* **96**, 2914-23.

Kozak. L. P., Kozak, U. C., and Clarke, G. T. (1991). Abnormal brown and white fat development in transgenic mice overexpressing glycerol 3-phosphate dehydrogenase. *Genes Dev* 5, 2256-64.

Krief. S., Lonnqvist, F., Raimbault, S., Baude, B., Van Spronsen, A., Arner, P., Strosberg. A. D., Ricquier, D., and Emorine, L. J. (1993). Tissue distribution of beta 3-adrenergic receptor mRNA in man. J *Clin invest* **91,** 344-9.

Lafontan, M.. and Berlan. M. (1993). Fat cell adrenergic receptors and the control of white and brown fat cell function. *J Lipid Res* **34,** 1057-91.

Lean, M. E., James, W. P., Jennings, G., and Trayhurn, P. (1986). Brown adipose tissue uncoupling protein content in human infants, children and adults. *Clin Sci* **71,** 291-7.

Lehmann, J. M., Moore, L. B., Smith-Oliver, T. A., Wilkison, W.O., Willson, T. M., and Kliewer, S. A. (1995). An antidiabetic thiazotidinedione is a high affinity ligand for peroxisome proliferator-activated receptor gamma (PPAR gamma). *J Biol Chem* **270*****,*** 12953-6.

Lin, F. T., and Lane, M. D. (1992). Antisense CCAAT/enhancer-binding protein RNA suppresses coordinate gene expression and triglyceride accumulation during differentiation of 3T3- L1 preadipocytes. *Genes Dev* **6,** 533-44.

Lin, F. T., and Lane. M. D. (1994). CCAAT/enhancer binding protein alpha is sufficient to initiate the 3T3- L1 adipocyte differentiation program. *Proc Natl Acad Sci USA* **91*****,*** 8757-61.

Loncar, D. (1991). Convertiblc adipose tissue in mice. *Cell Tissue Res* **266,** 149-61.

Lopez, J. M., Bennett. M. K., Sanchez, H. B., Rosenfeld, J. M., and Osborne, T. E. (1996). Sterol regulation of acetyl coenzyme A carboxylase: a mechanism for coordinate control of cellular lipid. *Proc Natl Acad Sci U S A* **93,** 1049-53.

Lowell, B. B., V, S. S., Hamann, A., Lawitts, J. A.. Himms-Hagen, J., Boyer, B. B., Kozak, L. P., and Flier, J. S. ( 1993). Development of obesity in transgenic mice after genetic ablation of brown adipose tissue. *Nature* **366,** 740-2.

MacDougald, O. A., Cornelius, P., Lin, F. T., Chen, S. S., and Lane, M. D. (1994). Glucocorticoids reciprocally regulate expression of the CCAAT/enhancer- binding protein alpha and delta genes in 3T3-L1 adipocytes and white adipose tissue. *J Biol Chem* **269,** 19041-7.

Mantzoros, C. S., Qu, D., Frederich, R. C., Susulic, V. S.. Lowell, B. B., Maratos-Flier, E., and Flier, J. S. (1996). Activation of beta(3) adrenergic receptors suppresses leptin expression and mediates a leptin-independent inhibition of food intake in mice. *Diabetes* **45,** 909-14.

Martin. G., Schoonjans, K., Lefebvre, A. M., Staels, B., and Auwerx, J. (1997). Coordinate regulation of the expression of the fatty acid transport protein and acyl-CoA synthetase genes by PPARalpha and PPARgamma activators. *J Biol Chem* **272,** 28210-7.

Maslowska, M., Sniderman, A. D., Germinario, R., and Cianflone, K. (1997). ASP stimulates glucose transport in cultured human adipocytes. *Int J Obes Relat Metab Disord* **21,** 261-6.

Miller, C. W., and Ntambi, J. M. (1996). Peroxisome proliferators induce mouse liver stearoyl-CoA desaturase I gene expression. *Proc Natl Acad Sci U S A* **93,** 9443-8.

Miura. N., Wanaka, A., Tohyama. M., and Tanaka, K. (1993). MFH-1, a new member of the fork head domain family, is expressed in developing mesenchyme. *FEBS Lett* **326,** 171-6.§

Muzzin, P., Revelli, J. P., Kuhne. F., Gocayne, J. D., McCombie, W. R., Venter, J. C., Giacobino, J. P., and Fraser, C. M. (1991). An adipose tissue-specific beta-adrenergic receptor. Molecular cloning and down-regulation in obesity. *J Biol Chem* **266,** 24053-8.

Nahmias, C., Blin, N., Elalouf, J. M., Mattei, M. G., Strosberg, A. D., and Emorine, L. J. (1991). Molecular characterization of the mouse beta 3-adrenergic receptor: relationship with the atypical receptor of adipocytes. *Embo J* **10,** 3721-7.

Nicholls, D. G., and Locke, R. M. (1984). Thermogenic mechanisms in brown fat. *Physiol Rev* **64,** 1-64.

Park, E. A., Roesler, W. J., Liu. J., Klemm, D. J., Gurney, A. L., Thatcher, J. D., Shuman, J., Friedman. A., and Hanson, R. W. (1990). The role of the CCAAT/enhancer-binding protein in the transcriptional regulation of the gene for phosphoenolpyruvate carboxykinase (GTP). *Mol Cell Biol* **10,** 6264-72.

Peraldi, P., Xu, M., and Spiegelman, B. M. (1997). Thiazolidinediones block tumor necrosis factor-alpha-induced inhibition of insulin signaling. *J Clin Invest* **100,** 1863-9.

Petruschke, T., and Hauner, H. (1993). Tumor necrosis factor-alpha prevents the differentiation of human adipocyte precursor cells and causes delipidation of newly developed fat cells. *J Clin Endocrinol Metab* **76**, 742-7.

Pierrou, S., Hellqvist, M., Samuelsson, L., Enerback, S., and Carlsson, P. (1994). Cloning and characterization of seven human forkhead proteins: binding site specificity and DNA bending. *Embo J* **13**, 5002-12.

Puigserver, P., Wu, Z., Park, C. W., Graves, R., Wright, M., and Spiegelman, B. M. (1998). A cold-inducible coactivator of nuclear receptors linked to adaptive thermogenesis. *Cell* **92**, 829-39.

Rehnmark, S., Nechad, M., Herron, D., Cannon, B., and Nedergaard, J. (1990). Alpha-and beta-adrenergic induction of the expression of the uncoupling protein thermogenin in brown adipocytes differentiated in culture. *J Biol Chem* **265**, 16464-71.

Ricquier. D., Bouillaud, F., Toumelin, P.. Mory, G., Bazin, R., Arch, J., and Penicaud, L. (1986). Expression of uncoupling protein mRNA in thermogenic or weakly thermogenic brown adipose tissue. Evidence for a rapid beta- adrenoreceptor-mediated and transcriptionally regulated step during activation of thermogenesis. *J Biol Chem* **261**, 13905-10.

Ross, S. R., Graves, R. A., Greenstein, A., Platt, K. A., Shyu, H. L., Mellovitz, B., and Spiegelman, B. M. (1990). A fat-specific enhancer is the primary determinant of gene expression for adipocyte P2 *in vivo. Proc Natl Acad Sci U S A* **87,** 9590-4.

Salmon, D. M., and Flatt, J. P. (1985). Effect of dietary fat content on the incidence of obesity among ad libitum fed mice. *Int J Obes* **9,** 443-9.

Samuelsson, L., Stromberg, K., Vikman, K., Bjursell, G., and Enerback, S. (1991). The CCAAT/enhancer binding protein and its role in adipocyte differentiation: evidence for direct involvement in terminal adipocyte development. *EMBO J* **10,** 3787-93.

Schoonjans, K., Peinado-Onsurbe, J., Lefebvre, A. M., Heyman, R. A., Briggs, M., Deeb, S., Staels, B., and Auwerx, J. (1996). PPARalpha and PPARgamma activators direct a distinct tissue-specific transcriptional response via a PPRE in the lipoprotein lipase gene. *EMBO J* **15**, 5336-48.

Sethi, J. K., and Hotamisligil, G. S. (1999). The role of TNF alpha in adipocyte metabolism. *Semin Cell Dca Biol* **10,** 19-29.

Shimomura, I., Hammer, R. E.. Richardson, J. A., Ikemoto, S., Bashmakov, Y., Goldstein, J. L., and Brown, M. S. (1998). Insulin resistance and diabetes mellitus in transgenic mice expressing nuclear SREBP-1c in adipose tissue: model for congenital generalized lipodystrophy. *Genes Dev* **12,** 3182-94.

Soloveva, V., Graves. R. A., Rasenick. M. M., Spiegelman. B. M., and Ross. S. R. (1997). Transgenic mice overexpressing the beta 1-adrenergic receptor in adipose tissue are resistant to obesity. *Mol Endocrinol* **11,** 27-38.

Susulic, V. S., Frederich, R. C., Lawitts, J.. Tozzo. E., Kahn, B. B., Harper, M. E., Himms-Hagen, J., Flier, J. S., and Lowell. B. B. (1995). Targeted disruption of the beta 3-adrenergic receptor gene. *J Biol Chem* **270,** 29483-92.

Tanaka, T., Yoshida, N., Kishimoto, T., and Akira, S. (1997). Defective adipocyte differentiation in mice lacking the C/EBPbeta and/or C/EBPdelta gene. *EMBO J* **16,** 7432-43.

Tontonoz, P., Hu, E., Devine, J., Beale, E. G., and Spiegelman, B. M. (1995). PPAR gamma 2 regulates adipose expression of the phosphoenolpyruvate carboxykinase gene. *Mol Cell Biol* **15,** 351-7.

Tontonoz, P., Hu, E., Graves, R. A., Budavari, A. I., and Spiegelman, B. M. (1994a), mPPAR gamma 2: tissue-specific regulator of an adipocyte enhancer. *Genes Dev* **8,** 1224-34.

Tontonoz, P., Hu, E., and Spiegelman, B. M. (1994b). Stimulation of adipogenesis in fibroblasts by PPAR gamma 2, a lipid- activated transcription factor [published erratum appears in Cell 1995 Mar 24;80(6):following 957]. *Cell* **79,** 1147-56.

Walsh, M. J., Sniderman, A. D., Cianflone, K., Vu, H., Rodriguez, M. A., and Forse, R. A. (1989). The effect of ASP on the adipocyte of the morbidly obese. *J Surg Res* **46,** 470-3.

Wang, N. D., Finegold, M. J., Bradley, A., Ou, C. N., Abdelsayed, S. V., Wilde, M. D., Taylor, L. R., Wilson, D. R., and Darlington, G. J. (1995). Impaired energy homeostasis in C/EBP alpha knockout mice. *Science* **269,** 1108-12.

Werman, A., Hollenberg, A., Solanes, G., Bjorbaek, C.. Vidal-Puig, A. J., and Flier, J. S. (1997). Ligand-independent activation domain in the N terminus of peroxisome proliferator-activated receptor gamma (PPARgamma). Differential activity of PPARgammal and -2 isoforms and intluence of insulin. *J Biol Chem* **272,** 20230-5.

W'ickelgren, 1. (1998). Obesity: how big a problem? [news]. *Science* **280,** 1364-7.

Winnier, G. E., Hargett, L., and Hogan. B. L. (1997). The winged helix transcription factor MFH 1 is required for proliferation and patterning of paraxial mesoderm in the mouse embryo. *Genes Dev* **11,** 926-40.

Wu, Z., Bucher, N. L., and Farmer, S. R. (1996). Induction of peroxisome proliferator-activated receptor gamma during the conversion of 3T3 fibroblasts into adipocytes is mediated by C/EBPbeta, C/EBPdelta, and glucocorticoids. *Mol Cell Biol* **16,** 4128-36.

Wu, Z., Puigserver, P., Andersson, U., Zhang, C., Adelmant, G., Mootha, V., Troy, A., Cinti, S., Lowell, B., Scarpulla, R. C., and Spiegelman, B. M. (1999a). Mechanisms controlling mitochondrial biogenesis and respiration through the thermogenic coactivator PGC-1. *Cell* **98,** 115-24.

Wu, Z., Rosen, E. D., Brun, R., Hauser, S., Adelmant, G., Troy, A. E., McKeon, C., Darlington, G. J., and Spiegelman, B. M. (1999b). Cross-regulation of C/EBP alpha and PPAR gamma controls the transcriptional pathway of adipogenesis and insulin sensitivity. *Mol Cell* **3,** 151-8.

Wu, Z., Xie, Y., Bucher, N. L., and Farmer, S. R. (1995). Conditional ectopic expression of C/EBP beta in NIH-3T3 cells induces PPAR gamma and stimulates adipogenesis. *Genes Dev* **9,** 2350-63.

Yeh, W. C., Cao, Z., Classon, M., and McKnight, S. L. (1995). Cascade regulation of terminal adipocyte differentiation by three members of the C/EBP family of leucine zipper proteins. *Genes Dev* **9,** 168-81.

Yubcro, P., Manchado, C., Cassard-Doulcier, A. M., Mampel, T., Vinas, O., Iglesias, R., Giralt, M.. and Villarroya, F. (1994). CCAAT/enhancer binding proteins alpha and beta are transcriptional activators of the brown fat uncoupling protein gene promoter. *Biochem Biophys Res Commun* **198,** 653-9.

Zhang, Y.. Proenca. R.. Maffei, M., Barone. M., Leopold, L., and Friedman, J. M. (1994). Positional cloning of the mouse obese gene and its human homologue [published erratum appears in Nature 1995 Mar 30;374(6521):479]. *Nature* **372,** 425-32.

## Claims

1. A method for identifying a compound useful for the treatment of obesity, said method comprising the steps
(i) contacting a test compound with the human *FOXC2* gene; and
(ii) determining whether said test compound activates the expression of the human *FOXC2* gene, such activation being indicative for a compound useful for the treatment of obesity.

2. A method for identifying a compound useful for the treatment of obesity, said method comprising the steps
(i) contacting a test compound with a polypeptide encoded by the human *FOXC2* gene; and
(ii) determining whether said test compound stimulates the biological activities of the said polypeptide, said biological activities comprising reducing total lipid content in a test animaL such stimulation being indicative for a compound useful for the treatment of obesity.

## Patentansprüche

1. Verfahren zur Identifizierung einer Verbindung, die bei der Behandlung von Fettleibigkeit nützlich ist, welches die Schritte umfasst
(i) in Kontakt bringen einer Testverbindung mit dem humanen FOXC2-Gen; und
(ii) bestimmen ob die Testverbindung die Expression des humanen FOXC2-Gens aktiviert, wobei die Aktivierung anzeigt, ob eine Verbindung bei der Behandlung von Fettleibigkeit nützlich ist.

2. Verfahren zur Identifizierung einer Verbindung, die bei der Behandlung von Fettleibigkeit nützlich ist, welches die Schritte umfasst
(i) in Kontakt bringen einer Testverbindung mit einem von dem humanen FOXC2 gecodiertem Polypeptide,
(ii) bestimmen, ob die Testverbindung die biologischen Aktivitäten des Polypeptides stimuliert, wobei die biologischen Aktivitäten die Reduktion des Gesamtlipidgehalts in einem Testtier umfassen, wobei eine derartige Stimulation anzeigt, ob eine Verbindung bei der Behandlung von Fettleibigkeit nützlich ist.

## Revendications

1. Procédé d'identification d'un composé utile pour le traitement d'obésité, ledit procédé comprenant les étapes de
(i) mise en contact d'un composé à tester avec le gène *FOXC2* humain; et
(ii) détermination pour savoir si ledit composé à tester active l'expression du gène *FOXC2* humain, ladite activation étant indicative pour un composé utile pour le traitement d'obésité.

2. Procédé d'identification d'un composé utile pour le traitement d'obésité, ledit procédé comprenant les étapes de
(i) mise en contact d'un composé à tester avec un polypeptide codé par le gène *FOXC2* humain; et
(ii) détermination pour savoir si ledit composé à tester stimule les activités biologiques dudit polypeptide, lesdites activités biologiques comprenant une réduction de la teneur totale en lipides dans un animal d'expérience, ladite stimulation étant indicative pour un composé utile pour le traitement d'obésité.
